(19) Europäisches Patentamt
European Patent Office
Office européen des brevets

(11) Veröffentlichungsnummer : **0 384 890 B1**

(12) **EUROPÄISCHE PATENTSCHRIFT**

(45) Veröffentlichungstag der Patentschrift : **09.03.94 Patentblatt 94/10**

(51) Int. Cl.$^5$ : **C07D 285/14,** A01N 43/82, C07D 417/12, C07F 7/08, A01N 55/00

(21) Anmeldenummer : **90810098.5**

(22) Anmeldetag : **13.02.90**

# (54) Mittel zum Schutz von Pflanzen gegen Krankheiten.

(30) Priorität : **21.02.89 CH 615/89**

(43) Veröffentlichungstag der Anmeldung : **29.08.90 Patentblatt 90/35**

(45) Bekanntmachung des Hinweises auf die Patenterteilung : **09.03.94 Patentblatt 94/10**

(84) Benannte Vertragsstaaten : **AT BE CH DE DK ES FR GB GR IT LI LU NL SE**

(56) Entgegenhaltungen : **EP-A- 0 313 512**
**GB-A- 1 176 799**

(73) Patentinhaber : **CIBA-GEIGY AG**
**Klybeckstrasse 141**
**CH-4002 Basel (CH)**

(72) Erfinder : **Kunz, Walter, Dr.**
**Buchenstrasse 9**
**CH-4104 Oberwil (CH)**
Erfinder : **Schurter, Rolf, Dr.**
**Holzmattstrasse 45**
**CH-4102 Binningen (CH)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 384 890 B1

## Beschreibung

Die vorliegende Erfindung betrifft neue Benz-1,2,3-thiadiazol-7-carbonsäureester der nachstehenden Formel I. Die Erfindung betrifft ferner die Herstellung dieser Substanzen sowie die als Wirkstoffe mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

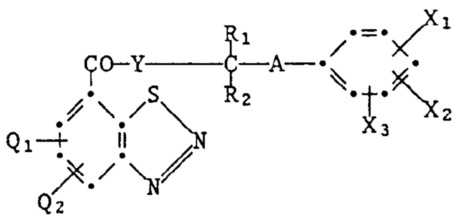

(I)

in welcher bedeuten:

| | |
|---|---|
| Y | Sauerstoff oder Schwefel; |
| $Q_1$ und $Q_2$ | unabhängig voneinander Wasserstoff oder Halogen; |
| $R_1$ und $R_2$ | unabhängig voneinander Wasserstoff oder $C_1$-$C_4$-Alkyl; |
| $X_1$ | Wasserstoff, Halogen, $C_1$-$C_4$-Alkyl, Trifluormethyl, $C_1$-$C_3$-Alkyl, im Alkylteil durch Halogen substituiertes $C_1$-$C_2$-Alkoxy, Nitro, Cyano, Dimethylamino, Phenyl, Phenoxy, durch Halogen und/oder $C_1$-$C_2$-Alkyl und/oder $C_1$-$C_2$-Alkoxy substituiertes Phenyl oder Phenoxy; |
| $X_2$ | Wasserstoff, Halogen oder Methyl; |
| $X_3$ | Wasserstoff oder Halogen; |
| | und |
| A | die Brückenglieder |

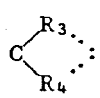 , 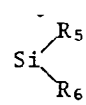 ,

Carbonyl, Ethenylen oder Ethinylen umfasst;

wobei

| | |
|---|---|
| $R_3$ | $C_1$-$C_6$-Alkyl, $C_3$-$C_6$-Alkenyl, Hydroxy, $C_1$-$C_6$-Alkoxy, O-C(O)-$C_1$-$C_4$-Alkyl, Carboxyl, $COOC_1$-$C_4$-Alkyl, Cyano oder Wasserstoff, mit der Massgabe, dass falls $R_3$ Wasserstoff darstellt, $R_1$, $R_2$ und $R_4$ nicht gleichzeitig Wasserstoff sind; |
| $R_4$ | $C_1$-$C_6$-Alkyl, $C_1$-$C_6$-Alkoxy oder Wasserstoff, mit der Massgabe, dass falls $R_4$ Wasserstoff darstellt, $R_1$, $R_2$ und $R_3$ nicht gleichzeitig Wasserstoff sind; |
| $R_5$ | $C_1$-$C_6$-Alkyl, $C_3$-$C_6$-Cycloalkyl, $C_1$-$C_6$-Alkoxy, Phenyl, oder durch Halogen oder Methoxy substituiertes Phenyl; |
| $R_6$ | $C_1$-$C_6$-Alkyl oder $C_1$-$C_6$-Alkoxy; |

oder ferner das Brückenglied

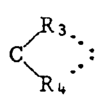 ,

welches zusammen auch einen gesättigten oder ungesättigten 3-7-gliedrigen Carbocyclus $W_1$ oder einen gesättigten oder ungesättigten 5-7-gliedrigen Heterocyclus $W_2$ mit 1 oder 2 Heteroatomen, die entweder Sauerstoff, Schwefel oder Stickstoff darstellen, bedeutet;

oder das Brückenglied

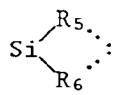 ,

welches zusammen einen gesättigten oder ungesättigten 3 bis 7-gliedrigen Heterocyclus $W_3$ mit maximal 2 Sauerstoff- oder Schwefelatomen als weitere Heteroatome bedeutet.

Als carbocyclische Ringe $W_1$ seien z.B. genannt Cyclopropyl, Cyclobutyl, Cyclopentyl, Cyclohexyl und Cycloheptyl oder Cycloheptenyl, vorzugweise Cyclopropyl, Cyclopentyl und Cyclohexyl. Als Heterocyclen $W_2$ kommen z.B. in Frage: 1,3-Dioxolan-2-yl, 1,3-Dioxolan-4-on-5-yl, Tetrahydrofuran-2-yl, Tetrahydrofuran-2-on-3-yl, 1,3-Dithiolan-2-yl, 1,3-Dioxan-2-yl, Tetrahydropyran-2-yl, Tetrahydro-1,3-oxazin-2-yl, Oxazolidin-2-yl, 1,3-Dithian-2-yl und 1,3-Dioxacyclohepten-4-2-yl.

Als Heterocyclen $W_3$ sind z.B. anzusehen 1-Sila-2,6-dioxacyclohexan, 1-Sila-2,5-dioxacyclopentan, 1-Sila-2,7-dioxacycloheptan, 1-Sila-2,5-dioxacyclohexan, 1-Sila-2-oxocyclopentan, 1-Sila-2-oxocyclohexan, 1-Sila-2,6-dithiacyclohexan, Sila-cyclopentan, Sila-cyclohexan, Sila-cycloheptan, 1-Sila-cyclohepten-4.

Die genannten cyclischen Reste $W_1$, $W_2$ und $W_3$ können unsubstituiert sein oder 1 bis 3 weitere Substituenten V tragen.

Hierbei kann V für $C_1$-$C_6$-Alkyl, $C_1$-$C_6$-Haloalkyl mit bis zu 5-Halogenatomen, vorzugsweise Fluor, $C_1$-$C_3$-Alkoxy, durch Halogen substituiertes $C_1$-$C_3$-Alkoxy, durch O oder S unterbrochenes oder durch Phenyl oder Phenoxy substituiertes $C_1$-$C_6$-Alkyl stehen.

Halogen bedeutet Fluor, Chlor, Brom oder Jod, bevorzugt Fluor und weiter in der Reihenfolge Chlor, Brom und Jod. Als Substituent in einzelnen Resten kann Halogen 1- bis 3-fach vertreten sein.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende Gruppen dar: Methyl, Ethyl sowie die Isomeren von Propyl, Butyl, Pentyl oder Hexyl, wie z.B. Isopropyl, Isobutyl, tert.-Butyl, sek.-Butyl oder Isopentyl.

Alkenyl steht z.B. für Propenyl-(1), Allyl, Butenyl-(1), Butenyl-(2) oder Butenyl-(3) und Alkinyl bedeutet z.B. Propinyl-(2), Butinyl-(1) oder Pentinyl-(4).

Aufgrund ihrer besonderen pflanzenschützenden Eigenschaften lassen sich die Verbindungen der Formel I in folgende Gruppen einteilen:

1. Verbindungen der Formel I, worin bedeuten:

Y Sauerstoff oder Schwefel;

$Q_1$ und $Q_2$ unabhängig voneinander Wasserstoff oder Halogen;

$R_1$ und $R_2$ unabhängig voneinander Wasserstoff oder $C_1$-$C_4$-Alkyl;

$X_1$ Wasserstoff, Halogen, $C_1$-$C_4$-Alkyl, Trifluormethyl, $C_1$-$C_3$-Alkyl, im Alkylteil durch Halogen substituiertes $C_1$-$C_2$-Alkoxy, Nitro, Cyano, Dimethylamino, Phenyl, Phenoxy, durch Halogen und/oder $C_1$-$C_2$-Alkyl und/oder $C_1$-$C_2$-Alkoxy substituiertes Phenyl oder Phenoxy;

$X_2$ Wasserstoff, Halogen oder Methyl;

$X_3$ Wasserstoff oder Halogen;

und

A die Brückenglieder

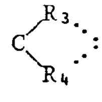 ,

Carbonyl, Ethenylen oder Ethinylen umfasst;

wobei

$R_3$ $C_1$-$C_6$-Alkyl, $C_3$-$C_6$-Alkenyl, Hydroxy, $C_1$-$C_6$-Alkoxy, O-C(O)-$C_1$-$C_4$-Alkyl, Carboxyl, $COOC_1$-$C_4$-Alkyl, Cyano oder Wasserstoff, mit der Massgabe, dass falls $R_3$ Wasserstoff darstellt, $R_1$, $R_2$ und $R_4$ nicht gleichzeitig Wasserstoff sind;

$R_4$ $C_1$-$C_6$-Alkyl, $C_1$-$C_6$-Alkoxy oder Wasserstoff, mit der Massgabe, dass falls $R_4$ Wasserstoff darstellt, $R_1$, $R_2$ und $R_3$ nicht gleichzeitig Wasserstoff sind;

oder ferner das Brückenglied

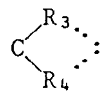 ,

welches zusammen auch einen gesättigten oder ungesättigten 3-7-gliedrigen Carbocyclus $W_1$ oder einen gesättigten oder ungesättigten 5-7-gliedrigen Heterocyclus $W_2$ mit 1 oder 2 Heteroatomen, die entweder Sauerstoff, Schwefel oder Stickstoff darstellen, bedeutet.

2. Verbindungen der Formel I, worin bedeuten:

Y Sauerstoff;
$Q_1$ und $Q_2$ unabhängig voneinander Wasserstoff, Fluor, Chlor oder Brom;
$R_1$ und $R_2$ unabhängig voneinander Wasserstoff, Methyl oder Ethyl;
$X_1$ Wasserstoff, Halogen, $C_1$-$C_4$-Alkyl, $C_1$-$C_3$-Alkoxy, Phenyl, Phenoxy, durch Halogen oder $C_1$-$C_2$-Alkyl substituiertes Phenyl oder substituiertes Phenoxy;
$X_2$ Wasserstoff oder Halogen;
$X_3$ Wasserstoff;
A die Brückenglieder

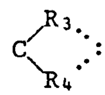 ,

Ethenylen, Ethinylen oder Carbonyl;
$R_3$ $C_1$-$C_6$-Alkyl, $C_3$-$C_6$-Alkenyl, Hydroxy, $C_1$-$C_6$-Alkoxy, Cyano oder $COOC_1$-$C_3$-Alkyl;
$R_4$ Wasserstoff, Methyl, Ethyl, Methoxy oder Ethoxy;
$W_1$ Cycloheptyl, Cyclohexyl, Cyclopentyl, Cyclobutyl oder Cyclopropyl;
$W_2$ 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

3. Verbindungen der Formel I, worin bedeuten:

Y Sauerstoff;
$Q_1$ und $Q_2$ unabhängig voneinander Wasserstoff oder Fluor;
$R_1$ und $R_2$ Wasserstoff oder Methyl;
$X_1$ Wasserstoff, Halogen, Methyl, Ethyl, Methoxy, Ethoxy, Phenyl, Phenoxy, mit Fluor, Chlor, Methyl oder Ethyl substituiertes Phenyl oder substituiertes Phenoxy;
$X_2$ Wasserstoff oder Halogen;
$X_3$ Wasserstoff;
A die Brückenglieder

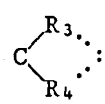 ,

Ethenylen oder Ethinylen;
$R_3$ $C_2$-$C_4$-Alkyl, $C_3$-$C_6$-Alkenyl, Hydroxy, O-$C_1$-$C_4$-Alkyl, Cyano oder $COOC_1$-$C_3$-Alkyl;
$R_4$ Wasserstoff, Methyl, Ethyl, Methoxy oder Ethoxy;
$W_1$ Cyclopentan oder Cyclohexan;
$W_2$ 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

4. Verbindungen der Formel I, worin bedeuten:

Y Sauerstoff;
$Q_1$ und $Q_2$ Wasserstoff;
$R_1$ und $R_2$ Wasserstoff;
$X_1$ Wasserstoff, Fluor, Chlor, Methyl, Methoxy, Phenyl, Phenoxy, mit Fluor oder Methyl substituiertes Phenyl oder substituiertes Phenoxy;
$X_2$ Wasserstoff, Fluor oder Chlor;
$X_3$ Wasserstoff;
A die Brückenglieder

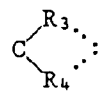 ,

Ethenylen oder Ethinylen;

$R_3$ Methyl, Ethyl, Allyl, Hydroxy, Methoxy oder Ethoxy;
$R_4$ Wasserstoff, Methyl, Ethyl, Methoxy oder Ethoxy;
$W_1$ Cyclopentan oder Cyclohexan;
$W_2$ 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

5. Verbindungen der Formel I, worin bedeuten:

Y Sauerstoff;
$Q_1$ und $Q_2$ Wasserstoff;
$R_1$ und $R_2$ Wasserstoff;
$X_1$ Wasserstoff, Fluor, Chlor oder Methyl;
$X_2$ Wasserstoff, Fluor oder Chlor;
$X_3$ Wasserstoff;
A das Brückenglied

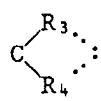 ;

$R_3$ Methyl, Ethyl, n-Propyl, i-Propyl, -n-Butyl, Allyl, Methoxy oder Ethoxy;
$R_4$ Wasserstoff, Methyl oder Ethyl.

Die folgenden Verbindungen zeichnen sich durch besonders vorteilhafte pflanzenschützende Eigenschaften aus:

[Benzo-1,2,3-thiadiazol-7-carbonyloxymethyl]-2'-4'-dichlorphenylketon (Verb. Nr. 1.2);
2-[Benzo-1,2,3-thiadiazol-7-carbonyloxymethyl]-1-phenyl-cyclopropan (Verb. Nr. 2.1);
1-[Benzo-1,2,3-thiadiazol-7-carbonyloxymethyl]-1-(4-methoxyphenyl)-cyclopentan (Verb. Nr. 2.6);
2-[Benzo-1,2,3-thiadiazol-7-carbonylthiomethyl]-1-phenyl-cyclopropan (Verb. Nr. 2.11);
3-[Benzo-1,2,3-thiadiazol-7-carbonyloxymethyl]-1-phenyl-1-propen (Verb. Nr. 3.1);
2-[Benzo-1,2,3-thiadiazol-7-carbonyloxy]-1-phenyl-n-propan (Verb. Nr. 4.1);
2-[Benzo-1,2,3-thiadiazol-7-carbonyloxy]-1-phenyl-ethanol (Verb. Nr. 4.16);
2-[Benzo-1,2,3-thiadiazol-7-carbonyloxy]-1-phenyl-propionsäuremethylester (Verb. Nr. 4.23);
1-[Benzo-1,2,3-thiadiazol-7-carbonyloxy]-2-phenyl-n-propan (Verb. Nr. 4.4);
1-[Benzo-1,2,3-thiadiazol-7-carbonyloxy]-2-phenyl-n-butan (Verb. Nr. 4.5);
1-[Benzo-1,2,3-thiadiazol-7-carbonyloxy]-2-(2',4'-dichlorphenyl)-n-butan (Verb. Nr. 4.6);
1-[6-Fluor-benzo-1,2,3-thiadiazol-7-carbonyloxy]-2-(2',4'-dichlorphenyl)-n-butan (Verb. Nr. 6.12).

Benzo-1,2,3-thiadiazole und ihre Verwendung gegen Pflanzenkrankheiten sind bereits bekannt, z.B. aus der nachveröffentlichten älteren europäischen Patenanmeldung mit der Veröffentlichungsnummer 313 512. Von diesen bekannten Verbindungen unterscheiden sich die Verbindungen der Formel I der vorliegenden Erfindung strukturell in charakteristischer Weise.

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mirkoorganismen mittels Blattapplikation oder Bodenapplikation als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekampfung mit chemischen Substanzen z.B. einerseits durch Schadigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der doppelten Wirkungsweise der erfindungsgemässen Verbindungen der Formel I, nämlich einerseits der direkten Bekämpfung der Pflanzenpathogene und andererseits der Erhöhung der allgemeinen Abwehrbereitschaft der mit diesen Wirkstoffen behandelten Pflanzen durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I eigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phyto-pathogenen Fungi wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden:

Pilze, wie z.B. Oomyceten (z.B. Plasmopara viticola, Phytophthora infestans, Peronospora tabacina, Pseudoperonospora, Bremia letucae), Fungi imperfecti (z.B. Colletotrichum lagenarium, Piricularia oryzae, Cercospora nicotinae), Ascomyceten (z.B. Venturia inaequalis); Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen unterschiedlicher Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemässen Verfahrens sind folgende Pflanzen anzusehen: Gurke, Tabak, Reben, Reis, Pfeffer, Kartoffeln, Tomaten, Weizen, Gerste, Birnen und Aepfel.

Die Verbindungen der Formel I werden wie folgt hergestellt:

a) Umsetzung eines aktivierten Carbonsäurederivates der Formel II

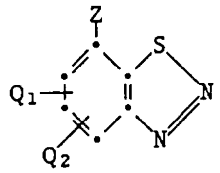

(II)

mit einem Alkohol oder Thiol der Formel III

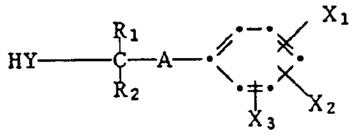

(III)

in Gegenwart einer Base gegebenenfalls mit einem Katalysator, wie z.B. 4-Dimethylaminopyridin, in inerten Lösungsmitteln bei Temperaturen von -20° bis 160°C, bevorzugt -10° bis 100°C, wobei Z die Reste Hal-CO,

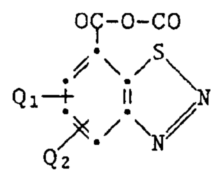

oder

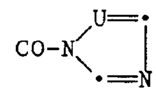

bedeutet, U, CH oder N und Hal Halogen darstellen.

b) Umsetzung der freien Säure der Formel II'

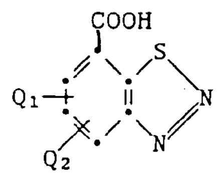

(II')

mit einem Alkohol oder Thiol der Formel III in Gegenwart eines sauren Katalysators, wie z.B. Schwefelsäure, gasförmigen Chlorwasserstoff oder Bromwasserstoff oder Bortrifluoridetherat, in einem inerten Lösungsmittel, wie z.B. Dioxan, Tetrahydrofuran oder Toluol, oder im Ueberschuss der zur Veresterung eingesetzten Alkohol- oder Thiol-Komponente der Formel III bei Temperaturen von 10° bis 180°C, vorzugsweise 0° bis 120°C.

Nach einem weiteren Verfahren findet die Herstellung von Verbindungen der Formel I, worin A die Carbonylgruppe darstellt, wie folgt statt:

Umsetzung der freien Carbonsäure der Formel II"

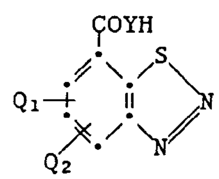

(II")

oder eines Alkalisalzes oder dessen Silbersalzes mit einem Alkylierungsmittel der Formel IV

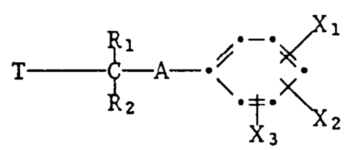

(IV)

gegebenenfalls unter Zusatz eines Katalysators, wie z.B. eines quaternären Ammoniumsalzes, unter Phasentransfer-Bedingungen (vgl. dazu W.E. Keller, Phasentransfer Reactions, Vol. 1, S. 68; Thieme-Verlag Stuttgart 1986), wobei T eine Abgangsgruppe, wie z.B. ein Halogen (F, Cl, Br, J), den Methansulfonyl-Rest, den Toluolsulfonyl-Rest oder den Trifluormethansulfonyl-Rest darstellt.

Nach einer speziellen Ausführungsform des Verfahrens zur Herstellung der Verbindungen der Formel I wird die freie Säure der Formel II' mit einem Alkylierungsmittel der Formel IV in Gegenwart von Kaliumfluorid in einem dipolaren aprotischen Lösungsmittel, wie z.B. N,N-Dimethylformamid, Dimethylsulfoxid, N-Methylpyrrolidon oder Tetramethylharnstoff, umgesetzt.

In den vorgängig beschriebenen Herstellungsverfahren besitzen die darin auftretenden Reste oder Symbole A, $Q_1$, $Q_2$, $R_1$, $R_2$, $X_1$, $X_2$ $X_3$ und Y die unter Formel I angegebenen Bedeutungen.

Als Basen kommen organische und anorganische Basen in Betracht, z.B. tertiäre Amine wie Trialkylamine (Trimethylamin, Triethylamin, Tripropylamin usw.), Pyridinbasen (Pyridin, 4-Dimethylaminopyridin, 4-Pyrrolidyilaminopyridin, Collidin), Oxide und Hydroxide, Carbonate und Hydrogencarbonate von Alkali- und Erdalkalimetallen sowie Alkaliacetate.

Als Reaktionsmedien in Anpassung an die jeweiligen Reaktionsbedingungen werden geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie

Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Ketone wie Aceton, Diethylketon, Methylethylketon; sowie Gemische solcher Lösungsmittel untereinander.

Die als Ausgangsverbindungen verwendeten Alklohole oder Thiole der Formel III sowie Verbindungen der Formel IV sind grösstenteils bekannt oder lassen sich nach dem Fachmann bekannten Methoden herstellen, wie z.B. nachfolgend beschrieben in:

J. Org. Chem. 33 2712 (1968); ibid 30, 23 (1964) ibid. 31, 876 (1966), ibid 39, 1265 (1974), "Organosilicon compounds", V. Ba ž ant et al. Academic Press New York 1965, Vol. 1 und 2; L. Zirngibl in "Progress in Drug Research Vol. 27, 253 (1983); Houben-Weyl, Vol. 9, S.3.

Die im Rahmen der Erfindung zur Anwendung gelangenden mikrobiziden Mittel zum Schutz von Pflanzen gegen Krankheiten, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-,Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände; verwendet werden.

Als oberflächenaktive Verbindungen kommen je nach Art des zu formulierenden Wirkstoffes der Formel I nicht-ionogene, kation-und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

## 1. Herstellungsbeispiele

### 1.1. Herstellung von 1-[Benzo-1,2,3-thiadiazol-7-carbonyloxymethyl]-1-phenylcyclopropan

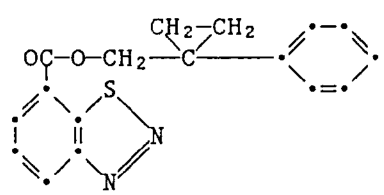

Eine Lösung von 5,0 g 1-Phenylcyclopropanmethanol, 6,1 ml Triethylamin und 200 mg 4-Dimethylaminopyridin in 50 ml Dichlormethan wird unter Kühlung bei maximal 15°C tropfenweise mit einer Lösung von 6,7 g Benzo-1,2,3-thiadiazol-7-carbonsäurechlorid in 33 ml Dichlormethan versetzt. Ueber Nacht wird bei Raumtemperatur bis zum Ende der Reaktion gerührt, mit Dichlormethan und Eiswasser versetzt, die wässrige Phase mit Dichlormethan extrahiert, die organische Phase mit Wasser und NaCl-Lösung gewaschen, über $Na_2SO_4$ getrocknet und eingedampft. Der Rückstand wurde an Kieselgel (Hexan-Essigsäureethylester 1:1) gereinigt und das erhaltene Produkt aus Diethylether/Hexan umkristallisiert. Es werden 7,5 g Kristalle vom Smp. 55-57°C erhalten.

### 1.2. Herstellung von [Benzo-1,2,3-thiadiazol-7-carbonyloxymethyl]-2',4'-dichlorphenyl-keton

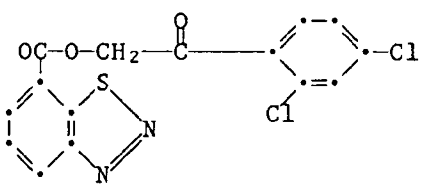

Eine Suspension von 4 g ω-Brom-2,4-dichloracetophenon und 1,9 g Kaliumfluorid in 15 ml N,N-Dimethylformamid wird portionenweise mit 4 g Benzo-1,2,3-thiadiazol-7-carbonsäure versetzt und über Nacht bei Raumtemperatur gerührt. Anschliessend wird mit Eiswasser versetzt, mit Dichlormethan extrahiert, die Extrakte mit Wasser gewaschen, getrocknet und eingedampft. Der Rückstand wird nach Entfernen des N,N-Dimethylformamids unter Vakuum (1,3 Pa/40°C) an Kieselgel (Hexan/Essigsäureethylester/Dichlormethan 6:2:1) gereinigt, wobei die Titelverbindung mit einem Smp. von 136°C resultiert.

1.3. <u>Herstellung von 3-[Benzo-1,2,3-thiadiazol-7-carbonyloxy]-1-phenyl-1-propen</u>

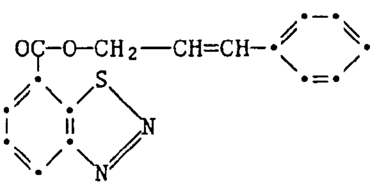

Zu einer Lösung von 2,3 g Zimtalkohol, 3,5 g Triethylamin und einer Spatelspitze 4-Dimethylaminopyridin in 13 ml Dichlormethan werden bei 15°C unter Kühlen und Rühren 3 g Benzo-1,2,3-thiadiazol-7-carbonsäurechlorid in 25 ml Dichlormethan zugetropft, 16 Stdn bei Raumtemperatur gerührt mit Eiswasser versetzt und die wässrige Phase mit Dichlormethan extrahiert. Die Extrakte werden mit Wasser gewaschen, über $Na_2SO_4$ getrocknet und eingedampft. Der feste Rückstand wird mit wenig Diethylether angerieben, wobei die Titelverbindung mit einem Smp. von 70-72°C resultiert.

Tabelle 1

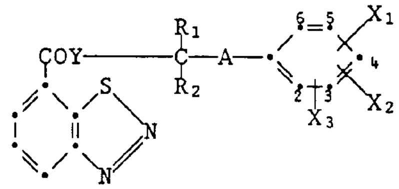

(A bedeutet 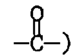)

| Verb. Nr. | Y | $R_1$ | $R_2$ | $X_1$ | $X_2$ | $X_3$ | physik.-Daten |
|---|---|---|---|---|---|---|---|
| 1.1 | O | H | H | 2-$OCH_3$ | H | H | Smp.159-161°C |
| 1.2 | O | H | H | 2-Cl | 4-Cl | H | Smp.136°C |
| 1.3 | O | H | H | 4-Cl | H | H | Smp.160°C |
| 1.4 | O | $CH_3$ | H | H | H | H | Smp.143-145°C |
| 1.5 | O | $CH_3$ | $CH_3$ | H | H | H | Smp.138-139°C |
| 1.6 | O | $C_2H_5$ | H | H | H | H | |
| 1.7 | O | $C_3H_7$-n | H | H | H | H | |
| 1.8 | O | $C_4H_9$-n | H | H | H | H | |
| 1.9 | S | $C_4H_9$-t | H | H | H | H | |
| 1.10 | S | H | H | H | H | H | |
| 1.11 | S | $C_3H_7$-n | $C_3H_7$-n | 2-Cl | 4-Cl | 6-Cl | |
| 1.12 | S | H | H | 2-F | 4-Cl | H | |
| 1.13 | O | H | H | 2-$CH_3$ | H | H | |
| 1.14 | O | H | H | 3-F | H | H | |
| 1.15 | O | H | H | 3-CN | H | H | |
| 1.16 | O | H | H | 3-$CF_3$ | H | H | |
| 1.17 | O | $C_4H_9$-n | $C_4H_9$-n | 4-$C_4H_9$-t | H | H | |
| 1.18 | O | H | H | 4-$OCF_3$ | H | H | |
| 1.19 | O | H | H | 2-$OCF_2CF_3$ | H | H | |

Fortsetzung Tabelle 1

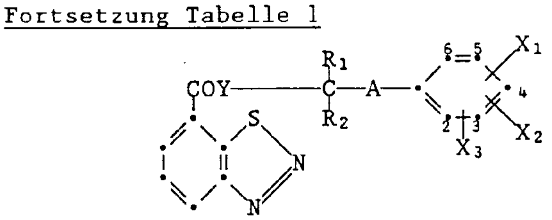

(A = bedeutet $-\overset{O}{\overset{\|}{C}}-$)

| Verb. Nr. | Y | $R_1$ | $R_2$ | $X_1$ | $X_2$ | $X_3$ | physik. Daten |
|---|---|---|---|---|---|---|---|
| 1.20 | S | H | H | $4-N(CH_3)_2$ | H | H | |
| 1.21 | O | $CH_3$ | H | $4-N(CH_3)_2$ | H | H | |
| 1.22 | O | $CH_3$ | $CH_3$ | $3-N(CH_3)_2$ | H | H | |
| 1.23 | O | H | H | $2-CH_3$ | 4-Cl | H | |
| 1.24 | O | H | H | 3-Cl | 5-Cl | H | |
| 1.25 | O | H | H | 2-Cl | 4-[4'-Cl Phenoxy] | H | Smp.122-124°C |
| 1.26 | O | H | H | 2-Br | 4-[4'-F-Phenoxy] | H | Smp.136-137°C |
| 1.27 | O | H | H | $2-CH_3$ | 4-Phenoxy | H | Smp.118-120°C |
| 1.28 | O | H | H | $2-CH_3$ | 4-[4'-Cl-Phenoxy] | H | Smp.135-136°C |
| 1.29 | S | H | H | 4-Phenoxy | H | H | |
| 1.30 | S | H | H | 4-Phenyl | H | H | |
| 1.31 | O | H | H | 3-Phenyl | H | H | |
| 1.32 | O | $CH_3$ | H | 4-Phenoxy | H | H | |
| 1.33 | O | $CH_3$ | $CH_3$ | 3-Phenyl | H | H | |
| 1.34 | O | $C_2H_5$ | H | $4-[4'OCH_3-$ Phenoxy] | H | H | |
| 1.35 | S | H | H | $4-[2'OC_2H_5-$ Phenoxy] | H | H | |
| 1.36 | O | H | H | $4-OC_2H_5$ | H | H | |
| 1.37 | O | $CH_3$ | $CH_3$ | 2-F | 4-F | 6-F | |
| 1.38 | O | $CH_3$ | $C_2H_5$ | 3-Cl | 4-Cl | H | |
| 1.39 | O | $n-C_4H_9$ | H | 4-Phenoxy | H | H | |
| 1.40 | O | H | H | $3-NO_2$ | H | H | |

Tabelle 2

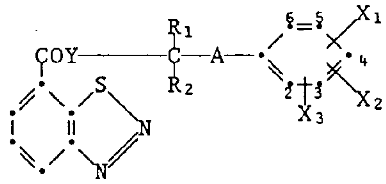

| Verb. Nr. | Y | $R_1$ | $R_2$ | $X_1$ | $X_2$ | $X_3$ | A | physik. Daten |
|---|---|---|---|---|---|---|---|---|
| 2.1 | O | H | H | H | H | H | 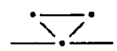 | Smp.55-57°C |
| 2.2 | O | H | H | 2-Cl | 4-Cl | H | 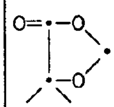 | Smp.156-158°C |
| 2.3 | O | H | H | 2-Cl | 4-Cl | H | 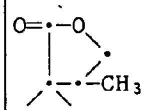 | Smp.201-203°C |
| 2.4 | O | H | H | $4-OCH_3$ | H | H | 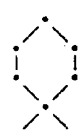 | Smp.105-107°C |
| 2.5 | O | H | H | 2-Cl | 4-Cl | H | 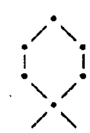 | Smp.130-134°C |
| 2.6 | O | H | H | $4-OCH_3$ | H | H | 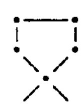 | Smp.62-64°C |
| 2.7 | O | H | H | 4-Br | H | H | 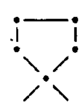 | Smp.103-104°C |
| 2.8 | O | H | H | 4-F | H | H | 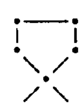 | Smp.103-104°C |
| 2.9 | O | H | H | $4-OC_2H_5$ | H | H | 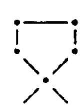 | Smp.64-68°C |
| 2.10 | O | H | H | $4-OC_2H_5$ | H | H | 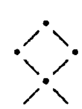 | Smp.100-102°C |
| 2.11 | S | H | H | H | H | H | 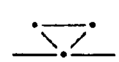 | |

Fortsetzung Tabelle 2

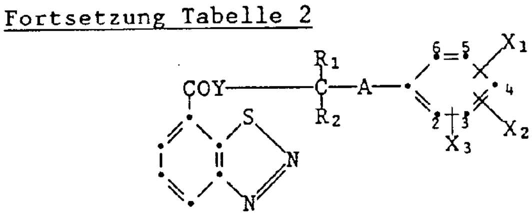

| Verb. Nr. | Y | $R_1$ | $R_2$ | $X_1$ | $X_2$ | $X_3$ | A | physik. Daten |
|---|---|---|---|---|---|---|---|---|
| 2.12 | O | H | H | 4-Cl | H | H | 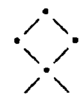 | |
| 2.13 | O | H | H | 2-Cl | 4-Cl | H | 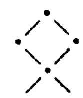 | |
| 2.14 | S | H | H | $-C_4H_9$-t | H | H | 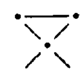 | |
| 2.15 | O | H | H | H | H | H | 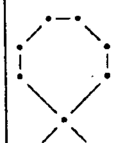 | |
| 2.16 | S | H | H | H | H | H | 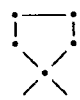 | |
| 2.17 | O | H | H | 4-Cl | H | H | 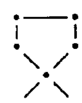 | |
| 2.18 | O | H | H | 2-Cl | 4-Cl | H | 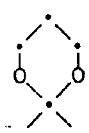 | |
| 2.19 | O | H | H | 2-F | H | H | 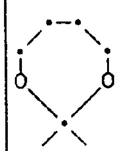 | |
| 2.20 | S | H | H | H | H | H | 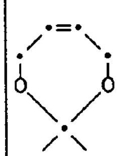 | |
| 2.21 | O | H | H | 2-Cl | 4-Cl | H | 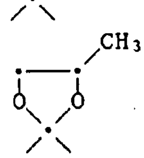 | |

Fortsetzung Tabelle 2

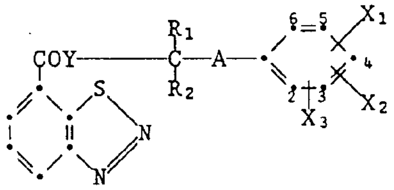

| Verb. Nr. | Y | $R_1$ | $R_2$ | $X_1$ | $X_2$ | $X_3$ | A | physik. Daten |
|---|---|---|---|---|---|---|---|---|
| 2.22 | O | H | H | 2-Cl | 4-Cl | H | 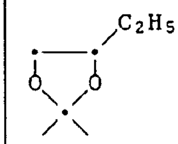 | |
| 2.23 | O | H | H | 4-Cl | H | H | 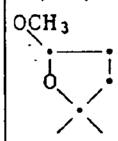 | |
| 2.24 | S | H | H | 2-Cl | 4-Cl | H | 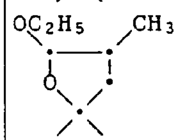 | |
| 2.25 | O | H | H | H | H | H | 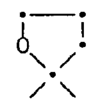 | |
| 2.26 | O | H | H | 2-Cl | 4-Cl | H | 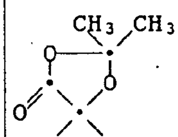 | |
| 2.27 | O | H | H | 2-Cl | 4-Cl | H | 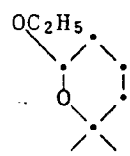 | |
| 2.28 | O | H | H | H | H | H | 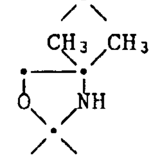 | |
| 2.29 | O | H | H | H | H | H | 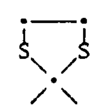 | |
| 2.30 | O | H | H | H | H | H | 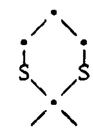 | |

Fortsetzung Tabelle 2

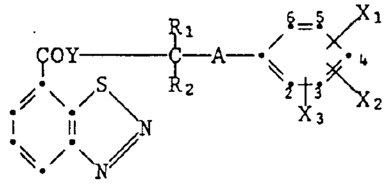

| Verb. Nr. | Y | $R_1$ | $R_2$ | $X_1$ | $X_2$ | $X_3$ | A | physik. Daten |
|---|---|---|---|---|---|---|---|---|
| 2.31 | S | H | H | 2-Cl | 4-Cl | H | 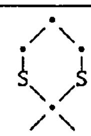 | |
| 2.32 | O | $CH_3$ | H | H | H | H | 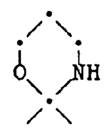 | |
| 2.33 | O | H | H | 2-Cl | 4-Cl | 6-Cl | 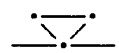 | |
| 2.34 | O | $CH_3$ | $CH_3$ | H | H | H | 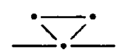 | |
| 2.35 | O | $C_4H_9$-n | H | 2-Cl | 4-Cl | H | 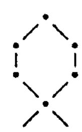 | |
| 2.36 | S | $C_3H_7$-i | H | H | H | H | 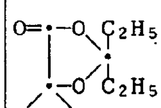 | |
| 2.37 | O | H | H | 2-Phenyl | H | H | 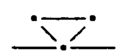 | |
| 2.38 | O | H | H | 3-O-Phenyl | H | H | 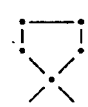 | |
| 2.39 | O | H | H | 2-$CH_3$ | 4-Cl-OPhenyl | H | 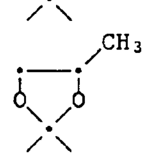 | |
| 2.40 | O | H | H | 2-$CH_3$ | 4-Cl-OPhenyl | H | 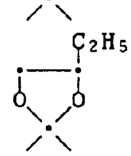 | |
| 2.41 | O | H | H | 2-Cl | 4-Cl-OPhenyl | H | 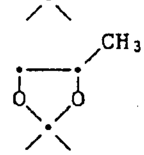 | |

Fortsetzung Tabelle 2

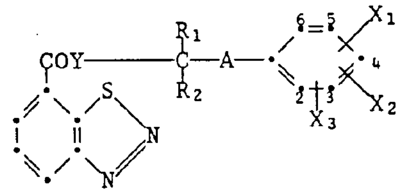

| Verb. Nr. | Y | $R_1$ | $R_2$ | $X_1$ | $X_2$ | $X_3$ | A | physik. Daten |
|---|---|---|---|---|---|---|---|---|
| 2.42 | S | $C_2H_5$ | H | 4-Phenyl | H | H | 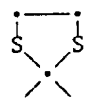 | |
| 2.43 | O | H | H | H | H | H | 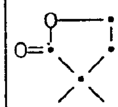 | |
| 2.44 | O | $C_3H_7$-n | $C_3H_7$-n | H | H | H | 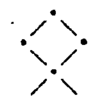 | |
| 2.45 | O | H | H | H | H | H | 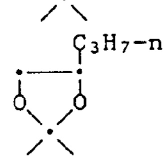 | |
| 2.46 | O | H | H | H | H | H | 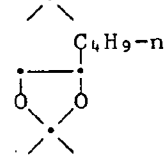 | |

Tabelle 3

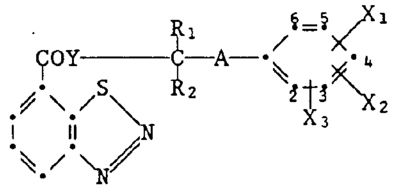

| Verb. Nr. | Y | $R_1$ | $R_2$ | $X_1$ | $X_2$ | $X_3$ | A | physik. Daten |
|---|---|---|---|---|---|---|---|---|
| 3.1 | O | H | H | H | H | H | -CH=CH- | Smp. 70-72°C |
| 3.2 | O | H | H | H | H | H | -C≡C- | |
| 3.3 | O | H | H | 2-$CH_3$ | H | H | -CH=CH- | |
| 3.4 | O | H | H | 2-$OCH_3$ | H | H | -CH=CH- | |
| 3.5 | O | H | H | 3-$NO_2$ | H | H | -CH=CH- | |
| 3.6 | O | H | H | 3-$CF_3$ | H | H | -CH=CH- | |
| 3.7 | O | H | H | 4-$OCF_3$ | H | H | -CH=CH- | |
| 3.8 | O | H | H | 4-$N(CH_3)_2$ | H | H | -CH=CH- | |
| 3.9 | O | $CH_3$ | H | 2-Cl | H | H | -CH=CH- | |
| 3.10 | O | $CH_3$ | H | 2-Cl | 4-Cl | H | -CH=CH- | |
| 3.11 | O | $CH_3$ | $CH_3$ | 2-Cl | 4-Cl | 6-Cl | -CH=CH- | |
| 3.12 | O | $C_4H_9$-n | $C_4H_9$-n | 3-Cl | 5-Cl | H | -CH=CH- | |
| 3.13 | S | H | H | H | H | H | -CH=CH- | |
| 3.14 | S | $C_4H_9$-n | H | 4-$OCH_3$ | H | H | -CH=CH- | |
| 3.15 | S | H | H | H | H | H | -C≡C- | |
| 3.16 | O | H | H | 3-CN | H | H | -CH=CH- | |
| 3.17 | O | H | H | 4-OPhenyl | H | H | -CH=CH- | |
| 3.18 | O | H | H | 4-Phenyl | H | H | -CH=CH- | |
| 3.19 | O | H | H | 3-Phenyl | H | H | -CH=CH- | |
| 3.20 | O | H | H | 2-$CH_3$ | 4-O-Phenyl | H | -CH=CH- | |

Tabelle 4

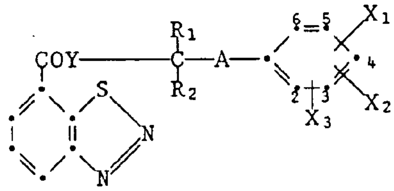

| Verb. Nr. | Y | $R_1$ | $R_2$ | $X_1$ | $X_2$ | $X_3$ | A | physik. Daten |
|---|---|---|---|---|---|---|---|---|
| 4.1 | O | $CH_3$ | H | H | H | H | $-CH_2-$ | $n_D^{25}$ 1.5932 |
| 4.2 | O | $CH_3$ | $CH_3$ | H | H | H | $-CH_2-$ | |
| 4.3 | O | $CH_3$ | $CH_3$ | H | H | H | $-CH(CH_3)-$ | |
| 4.4 | O | H | H | H | H | H | $-CH(CH_3)-$ | $n_D^{25}$ 1.6000 |
| 4.5 | O | H | H | H | H | H | $-CH(C_2H_5)-$ | $n_D^{25}$ 1.5958 |
| 4.6 | O | H | H | 2-Cl | 4-Cl | H | $-CH-(C_3H_7-n)-$ | $n_D^{25}$ 1.5876 |
| 4.7 | O | H | H | H | H | H | $-CH-(C_4H_9-n)-$ | |
| 4.8 | S | H | H | H | H | H | $-CH-(C_4H_9-i)-$ | |
| 4.9 | O | H | H | H | H | H | $-CH-(C_4H_9-t)-$ | |
| 4.10 | O | H | H | H | H | H | -CH-(n-Pentyl)- | |
| 4.11 | S | H | H | H | H | H | -CH-(n-Hexyl)- | |
| 4.12 | O | H | H | 2-Cl | 4-Cl | H | -CH-(Allyl) | |
| 4.13 | O | $C_4H_9-n$ | H | 2-Cl | H | H | -CH-(2-Butenyl)- | |
| 4.14 | S | H | H | 2-F | 4-F | H | $-C(CH_3)_2-$ | |
| 4.15 | O | H | H | 2-Cl | 4-Cl | H | $-C(CN)(C_4H_9-n)-$ | |
| 4.16 | O | H | H | H | H | H | -CH(OH)- | |
| 4.17 | O | H | H | 2-Cl | 4-Cl | H | -C(OH)(n-Pr)- | |
| 4.18 | O | H | H | 2-Cl | 4-Cl | 6-Cl | $-CH(OCH_3)-$ | |
| 4.19 | O | H | H | 2-Cl | 4-Cl | H | $-C(OCH_3)(C_3H_7-n)-$ | |
| 4.20 | S | $C_3H_7-n$ | H | H | H | H | -CH(n-Hexyl)- | |
| 4.21 | O | H | H | 2-Cl | 4-Cl | H | -CH(OH)- | |

Fortsetzung Tabelle 4

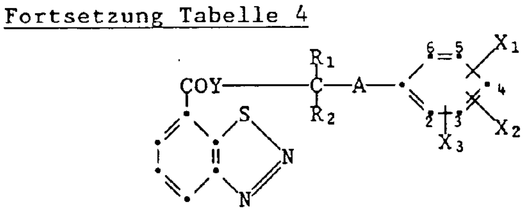

| Verb. Nr. | Y | $R_1$ | $R_2$ | $X_1$ | $X_2$ | $X_3$ | A | physik. Daten |
|---|---|---|---|---|---|---|---|---|
| 4.22 | O | H | H | H | H | H | $-CH(COOH)-$ | |
| 4.23 | O | H | H | H | H | H | $-CH(COOCH_3)-$ | |
| 4.24 | O | H | H | H | H | H | $-CH(COOC_4H_9-n)-$ | |
| 4.25 | O | H | H | H | H | H | $-CH(COOC_3H_7-n)-$ | |
| 4.26 | O | H | H | 2-F | H | H | $-CH[OC(O)CH_3]-$ | |
| 4.27 | O | H | H | H | H | H | $-CH[OC(O)C_4H_9-i]-$ | |
| 4.28 | O | $CH_3$ | H | H | H | H | $-C(OCH_3)_2-$ | |
| 4.29 | O | H | H | H | H | H | $-C(OC_2H_5)_2-$ | |
| 4.30 | O | H | H | H | H | H | $-C(OC_4H_9-n)_2-$ | |
| 4.31 | O | H | H | H | H | H | $-C(O-sec-C_6H_{13})_2-$ | |
| 4.32 | O | H | H | 2-Cl | 4-Cl | H | $-CH(COOCH_3)-$ | |
| 4.33 | O | H | H | 2-Cl | 4-Cl | H | $-CH(COO-C_3H_7-n)-$ | |
| 4.34 | O | H | H | 2-Cl | 4-Cl | H | $-CH[OC(O)CH_3]-$ | |
| 4.35 | O | H | H | 2-Cl | 4-F | H | $-C(OC_4H_9-n)_2-$ | |
| 4.36 | S | $C_4H_9-n$ | H | H | H | H | $-CH(COOC_2H_5)-$ | |
| 4.37 | O | $CH_3$ | $CH_3$ | H | H | H | $-C(OC_2H_5)_2-$ | |
| 4.38 | O | H | H | 2-Phenyl | H | H | $-CH(COOC_3H_7-n)-$ | |
| 4.39 | O | H | H | 4-Phenyl | H | H | $-C(OH)(COOCH_3)-$ | |
| 4.40 | O | H | H | 2-Cl | 4-Cl | H | $C(OH)(COOC_3H_7-n)-$ | |
| 4.41 | O | H | H | 4-OPhenyl | H | H | $-C(OC_3H_7-n)_2-$ | |
| 4.42 | S | H | H | $2-CH_3$ | $4-NO_2$ | H | $CH(COOCH_3)-$ | |

Tabelle 5

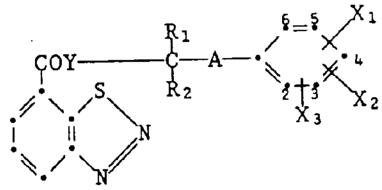

| Verb. Nr. | Y | $R_1$ | $R_2$ | $X_1$ | $X_2$ | $X_3$ | A | physik. Daten |
|---|---|---|---|---|---|---|---|---|
| 5.1 | O | H | H | H | H | H | 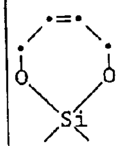 | |
| 5.2 | O | H | H | H | H | H | 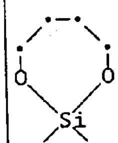 | |
| 5.3 | O | $CH_3$ | H | 2-Cl | 4-Cl | H | 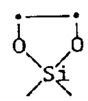 | |
| 5.4 | O | H | H | 4-F | H | H | 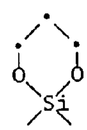 | |
| 5.5 | S | H | H | 4-$OCH_3$ | H | H | 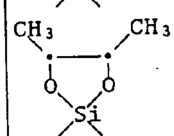 | |
| 5.6 | O | $C_2H_5$ | $C_2H_5$ | 2-Cl | 4-Cl | 6-Cl | 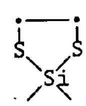 | |
| 5.7 | O | $C_4H_9$-n | H | H | H | H | 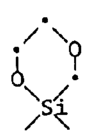 | |
| 5.8 | O | $CH_3$ | H | H | H | H | 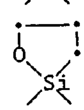 | |
| 5.9 | O | H | H | H | H | H | 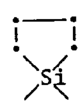 | |

Fortsetzung Tabelle 5

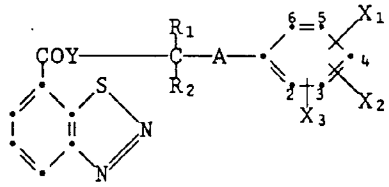

| Verb. Nr. | Y | $R_1$ | $R_2$ | $X_1$ | $X_2$ | $X_3$ | A | physik. Daten |
|---|---|---|---|---|---|---|---|---|
| 5.10 | O | H | H | 4-F | H | H | 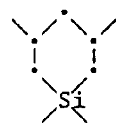 | |
| 5.11 | O | $CH_3$ | H | 4-F | H | H | 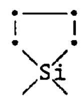 | |
| 5.12 | O | H | H | H | H | H | $-Si(OC_2H_5)_2-$ | |
| 5.13 | O | H | H | H | H | H | $-(CH_3)Si(OCH_3)-$ | |
| 5.14 | O | H | H | H | H | H | -(Phenyl)Si($OC_2H_5$)- | |
| 5.15 | S | H | H | H | H | H | -(Phenyl)Si($CH_3$)- | |
| 5.16 | S | H | H | H | H | H | -(Phenyl)Si($C_3H_7$-n)- | |
| 5.17 | O | H | H | 4-F | H | H | -(2'-F-Phenyl)Si($CH_3$)- | |
| 5.18 | O | H | H | 4-F | H | H | -(4'-F-Phenyl)Si(OH)- | |
| 5.19 | O | $CH_3$ | H | H | H | H | $-Si(OC_4H_9-n)_2-$ | |

Tabelle 6

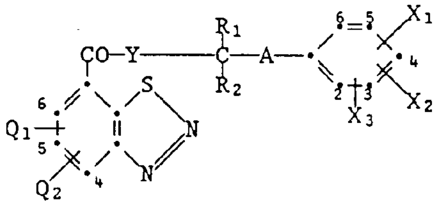

| Verb. Nr. | Y | $Q_1$ | $Q_2$ | $R_1$ | $R_2$ | $X_1$ | $X_2$ | $X_3$ | A | physik. Daten |
|---|---|---|---|---|---|---|---|---|---|---|
| 6.1 | O | 5-Cl | H | H | H | 4-Cl | H | H | -C(O)- | |
| 6.2 | O | 5-F | H | $CH_3$ | H | 2-Cl | 4-Cl | H | -C(O)- | Oel |
| 6.3 | O | 6-F | H | H | H | H | H | H | -C(O)- | |
| 6.4 | S | 4-F | H | $CH_3$ | $CH_3$ | H | H | H | -CH(OH)- | |
| 6.5 | O | 5-Br | H | H | H | H | H | H | -C(O)- | |
| 6.6 | O | 5-F | H | H | H | H | H | H | 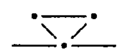 | |
| 6.7 | O | 5-F | 6-F | H | H | H | H | H | 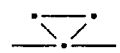 | |
| 6.8 | O | 5-J | H | $C_2H_5$ | H | 2-Cl | 4-Cl | 6-Cl | 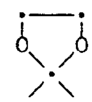 | |
| 6.9 | O | 4-F | 5-F | H | H | H | H | H | 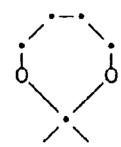 | |
| 6.10 | O | 6-Cl | H | H | H | 2-$CH_3$ | 4-O-Phenyl | H | -CH=CH- | |
| 6.11 | O | 5-F | H | H | H | H | H | H | -C≡H- | |
| 6.12 | O | 6-F | H | H | H | 2-Cl | 4-Cl | H | $-CH(C_3H_7-n)-$ | $n_D^{25}$ 1.5817 |
| 6.13 | S | 4-F | H | $CH_3$ | $CH_3$ | H | H | H | $-C(OH)(C_4H_9-n)-$ | |
| 6.14 | O | 5-J | H | H | H | 2-Cl | 4-Cl | H | $-C(OH)(COOCH_3)-$ | |
| 6.15 | S | 4-F | H | $C_4H_9$-n | H | H | H | H | 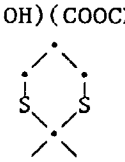 | |
| 6.16 | O | 4-F | H | H | H | 2-Cl | 4-Cl | H | $-CH(C_3H_7-n)-$ | |
| 6.17 | O | 4-F | H | H | H | H | H | H | $-CH(CH_3)-$ | |

Formulierungsbeispiele für Wirkstoffe der Formel I (% = Gewichtsprozent)

| 2.1 Spritzpulver | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

| 2.2 Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.3 Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

2.4 Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5 Umhüllungs-Granulat

| | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensionskonzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200 ppm). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C bis 23°C weitergeführt.

Die Beurteilung der Schutzwirkung erfolgt aufgrund des Pilzbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C weitergeführt.

Die Beurteilung der Schutzwirkung erfolgt aufgrund des Pilzbefalls 7-8 Tage nach der Infektion.

c) Gurkenpflanzen werden nach 2-wöchiger Anzucht mit einer aus Spritzpulver des Wirkstoffes hergestellten Spritzbrühe besprüht (Konzentration: 200 ppm).

Nach 3 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchigkeit und bei 22° bis 23°C weitergeführt.

Die Beurteilung der Schutzwirkung erfolgt aufgrund des Pilzbefalls 7-8 Tage nach der Infektion.

Verbindungen aus den Tabellen 1 bis 6 zeigten in den Tests (a) und (b) gute Wirkung. So reduzierten z.B. die Verbindungen 1.2, 1.3, 1.4, 1.28, 2.1, 2.2, 2.5, 3.1 und 4.6 den Pilzbefall auf 0 bis 20 %. Unbehandelte jedoch infizierte Kontrollpflanzen wiesen dagegen einen Colletotrichum-Befall von 100 % auf.

Beispiel 3.2: Wirkung gegen Puccinia graminis auf Weizen

a) Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) Zu Weizenpflanzen wird 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus den Tabellen 1 bis 6 zeigten gegen Puccinia-Pilze eine gute Wirkung. So reduzierten z.B. die Verbindungen 1.1, 1.27, 2.2, 2.10, 3.1 und 4.6 den Pilzbefall auf 0 bis 20 %. Unbehandelte jedoch infizierte Kontrollpflanzen wiesen dagegen einen Puccinia-Befall von 100 % auf.

Beispiel 3.3: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20°C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen 1 bis 6 zeigten gegen den Phytophthora-Pilz eine gute Schutzwirkung. So reduzierten z.B. die Verbindungen 1.1, 1.2, 1.3. 1.4, 1.5, 1.6, 1.25, 1.26, 1.28, 2.1, 2.5, 2.6, 3.1 und 4.6 den Pilzbefall auf 0 bis 20 %. Unbehandelte jedoch infizierte Kontrollpflanzen wiesen dagegen einen 100 %igen Phytophthora-Befall auf.

Beispiel 3.4: Wirkung gegen Piricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall beurteilt.

b) Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24°C wird der Pilzbefall beurteilt.

Reispflanzen, die mit einer Spritzbrühe behandelt wurden, die als Aktivsubstanz eine Verbindung aus den Tabellen 1 bis 6 enthielt, zeigten im Vergleich zu unbehandelten Kontrollpflanzen (100 % Befall) nur geringen Pilzbefall. So reduzierten z.B. im Test (a) die Verbindungen 1.1, 1.2, 1.3, 1.5, 1.25, 1.27, 2.1, 2.2, 2.3, 2.5, 2.8, 3.1 und 4,6 und im Test b) die Verbindungen 1.2, 1.3, 2.1, 2.5, 2.6, 2.8 und 2.10 den Befall auf 0 bis 20 %.

Beispiel 3.5: Wirkung gegen Xanthomonas oryzae auf Reis (Oryza sativa)

a) Reispflanzen der Sorte "Calora" oder "S6" werden nach 3-wöchiger Anzucht im Gewächshaus mit der Prüfsubstanz in Form einer Spritzbrühe (0,02 % Aktivsubstanz) besprüht. Nach eintägigem Antrocken dieses Spritzbelags werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der residual Wirksamkeit der Prüfsubstanz.

b) Reispflanzen der Sorte "Calora" oder "S6" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der systemischen Wirksamkeit der Prüfsubstanz.

Verbindungen aus den Tabellen 1 bis 6 zeigten eine gute Schutzwirkung gegen Xanthomonas oryzae. So reduzierten z.B. im Test (a) die Verbindungen 2.1 und 3.1 sowie im Test (b) die Verbindung 3.1, den Bakterienbefall auf 0 bis 20 %. Unbehandelte jedoch infizierte Kontrollpflanzen wiesen dagegen einen Krankheitsbefall von 100 % auf.

Beispiel 3.6: Wirkung gegen Xanthomonas vesicatoria auf Paprika (Capsicum annuum)

a) Paprikapflanzen der Sorte "California Wonder" werden nach 3-wöchiger Anzucht im Gewächshaus mit der Prüfsubstanz in Form einer Spritzbrühe (0,02 % Aktivsubstanz) besprüht. Nach eintägigem Antrocknen dieses Spritzbelages werden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der residualen Wirksamkeit der Prüfsubstanz.

b) Paprikapflanzen der Sorte "California Wonder" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der systemischen Wirksamkeit der Prüfsubstanz.

Verbindungen aus den Tabellen 1 bis 6 zeigten eine gute Schutzwirkung gegen Xanthomonas vesicatoria. So reduzierten z.B. im Test (a) die Verbindungen 2.1, 2.2 und 3.1 und im Test (b) die Verbindung 3.1 den Bakterienbefall auf 0 bis 20 %. Unbehandelte jedoch infizierte Kontrollpflanzen wiesen dagegen einen Krankheitsbefall von 100 % auf.

Beispiel 3.7: Wirkung gegen Pseudomonas tomato an Tomatenpflanzen

a) Tomatenpflanzen werden nach 3-wöchiger Anzucht mit einer aus Spritzpulver des Wirkstoffes hergestellten Spritzbrühe durch Blattapplikation behandelt (Konzentration: 200 ppm). Nach 3,5 Wochen werden die Pflanzen mit einer Bakteriensuspension ($10^8$ Bakterien/ml) inokuliert und für 6 Tage bei hoher Luftfeuchtigkeit und bei einer Temperatur von 25°C inkubiert. Die Beurteilung der Schutzwirkung erfolgt aufgrund des Bakterienbefalls 7-8 Tage nach Inokulation.

Unbehandelte aber infizierte Kontrollpflanzen weisen in diesem Test einen Befall von 100 % auf.

Verbindungen aus den Tabellen 1 bis 6 zeigten eine gute Schutzwirkung gegen Pseudomonas tomato. So blieben Pflanzen, die z.B. mit der Verbindung 2.1 oder 3.1 behandelt wurden, weitgehend frei von Pseudomonas (Befall: 20 bis 0 %).

b) Tomatenpflanzen werden nach 3-wöchiger Anzucht mit einer aus Spritzpulver des Wirkstoffes hergestellten Spritzbrühe durch Bodenapplikation behandelt (Konzentration: 60 ppm bezogen auf das Bodenvolumen). Nach 3,5 Wochen werden die Pflanzen mit Bakteriensuspension ($10^8$ Bakterien/ml) inokuliert und für 6 Tage bei hoher Luftfeuchtigkeit und bei einer Temperatur von 25°C inkubiert.

Die Beurteilung der Schutzwirkung erfolgt aufgrund des Bakterienbefalls 7-8 Tage nach Inokulation. Unbehandelte aber infizierte Kontrollpflanzen weisen in diesem Test einen Befall von 100 % auf.

Verbindungen aus den Tabellen 1 bis 6 bewirken eine gute Wirkung gegen Pseudomonas tomato. So blieben Pflanzen, die z.B. mit der Verbindung 3.1 behandelt wurden, fast völlig frei von Pseudomonas (Befall: 20 bis 0 %).

Beispiel 3.8: Wirkung gegen Cercospora nicotianae an Tabakpflanzen

a) Tabakpflanzen (8 Wochen alt) werden mit einer formulierten Lösung des Wirkstoffs besprüht (Konzentration: 200 ppm). Vier Tagen nach der Behandlung werden die Pflanzen mit einer Sporensuspension von Cercospora nicotianae ($10^5$ Sporen/ml) inokuliert und für 5 Tage bei hoher Luftfeuchtigkeit und einer Temperatur von 22°-25°C inkubiert. Die Inkubation wird dann bei normaler Luftfeuchtigkeit und bei 20°-22°C weitergeführt.

b) Tabakpflanzen (8 Wochen alt) wurden mit einer formulierten Lösung des Wirkstoffs durch Bodenapplikation behandelt (Konzentration: 0,002 % Aktivsubstanz). Nach 4 Tagen wurden die Pflanzen mit einer Sporensuspension von Cercospora nicotianae ($10^5$ Sporen/ml) inokuliert und für 5 Tage bei hoher Luftfeuchtigkeit und einer Temperatur von 22°-25°C inkubiert. Die Inkubation wurde dann bei normaler Luftfeuchtigkeit und bei 20°-22°C weitergeführt.

Die Beurteilung der Symptome in den Tests (a) und (b) erfolgt aufgrund des Pilzbefalls 12 bis 14 Tage nach der Infektion.

Die Kontrollpflanzen zeigten einen Befall von 100 %. Pflanzen, welche in den Tests (a) und (b) mit der Verbindung 2.1 behandelt wurden, zeigten einen Befall von 0-20 %.

## Patentansprüche

### Patentansprüche für folgende Vertragsstaaten : AT, BE, CH, DE, DK, FR, GB, GR, IT, LI, LU, NL, SE

1. Verbindungen der Formel I

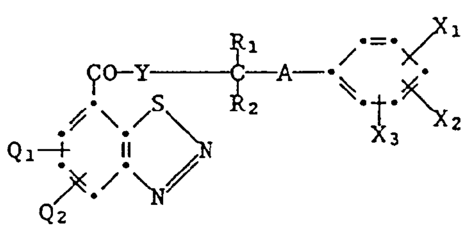

(I)

in welcher bedeuten:

| | |
|---|---|
| Y | Sauerstoff oder Schwefel; |
| $Q_1$ und $Q_2$ | unabhängig voneinander Wasserstoff oder Halogen; |
| $R_1$ und $R_2$ | unabhängig voneinander Wasserstoff oder $C_1$-$C_4$-Alkyl; |
| $X_1$ | Wasserstoff, Halogen, $C_1$-$C_4$-Alkyl, Trifluormethyl, $C_1$-$C_3$-Alkyl, im Alkylteil durch Halogen substituiertes $C_1$-$C_2$-Alkoxy, Nitro, Cyano, Dimethylamino, Phenyl, Phenoxy, durch Halogen und/oder $C_1$-$C_2$-Alkyl und/oder $C_1$-$C_2$-Alkoxy substituiertes Phenyl oder Phenoxy; |
| $X_2$ | Wasserstoff, Halogen oder Methyl; |
| $X_3$ | Wasserstoff oder Halogen; |
| | und |
| A | die Brückenglieder |

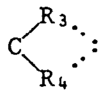 , 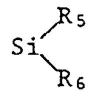 ,

Carbonyl, Ethenylen oder Ethinylen umfasst;
wobei

$R_3$ — $C_1$-$C_6$-Alkyl, $C_3$-$C_6$-Alkenyl, Hydroxy, $C_1$-$C_6$-Alkoxy, O-C(O)-$C_1$-$C_4$-Alkyl, Carboxyl, COOC$_1$-$C_4$-Alkyl, Cyano oder Wasserstoff, mit der Massgabe, dass falls $R_3$ Wasserstoff darstellt, $R_1$, $R_2$ und $R_4$ nicht gleichzeitig Wasserstoff sind;

$R_4$ — $C_1$-$C_6$-Alkyl, $C_1$-$C_6$-Alkoxy oder Wasserstoff, mit der Massgabe, dass falls $R_4$ Wasserstoff darstellt, $R_1$, $R_2$ und $R_3$ nicht gleichzeitig Wasserstoff sind;

$R_5$ — $C_1$-$C_6$-Alkyl, $C_3$-$C_6$-Cycloalkyl, $C_1$-$C_6$-Alkoxy, Phenyl, oder durch Halogen oder Methoxy substituiertes Phenyl;

$R_6$ — $C_1$-$C_6$-Alkyl oder $C_1$-$C_6$-Alkoxy;

oder ferner das Brückenglied

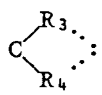 ,

welches zusammen auch einen gesättigten oder ungesättigten 3-7-gliedrigen Carbocyclus $W_1$ oder einen gesattigten oder ungesättigten 5-7-gliedrigen Heterocyclus $W_2$ mit 1 oder 2 Heteroatomen, die entweder Sauerstoff, Schwefel oder Stickstoff darstellen, bedeutet;

oder das Brückenglied

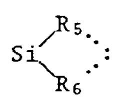 ,

welches zusammen einen gesättigten oder ungesättigten 3 bis 7-gliedrigen Heterocyclus $W_3$ mit maximal 2 Sauerstoff- oder Schwefelatomen als weitere Heteroatome bedeutet.

**2.** Verbindungen der Formel I gemäss Anspruch 1, worin bedeuten:

Y — Sauerstoff oder Schwefel;

$Q_1$ und $Q_2$ — unabhängig voneinander Wasserstoff oder Halogen;

$R_1$ und $R_2$ — unabhängig voneinander Wasserstoff oder $C_1$-$C_4$-Alkyl;

$X_1$ — Wasserstoff, Halogen, $C_1$-$C_4$-Alkyl, Trifluormethyl, $C_1$-$C_3$-Alkyl, im Alkylteil durch Halogen substituiertes $C_1$-$C_2$-Alkoxy, Nitro, Cyano, Dimethylamino, Phenyl, Phenoxy, durch Halogen und/oder $C_1$-$C_2$-Alkyl und/oder $C_1$-$C_2$-Alkoxy substituiertes Phenyl oder Phenoxy;

$X_2$ — Wasserstoff, Halogen oder Methyl;

$X_3$ — Wasserstoff oder Halogen;

und

A — die Brückenglieder

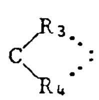 ,

Carbonyl, Ethenylen oder Ethinylen umfasst;
wobei

$R_3$ — $C_1$-$C_6$-Alkyl, $C_3$-$C_6$-Alkenyl, Hydroxy, $C_1$-$C_6$-Alkoxy, O-C(O)-$C_1$-$C_4$-Alkyl, Carboxyl, COOC$_1$-$C_4$-Alkyl, Cyano oder Wasserstoff, mit der Massgabe dass falls $R_3$ Wasserstoff darstellt, $R_1$, $R_2$ und $R_4$ nicht gleichzeitig Wasserstoff sind;

$R_4$ — $C_1$-$C_6$-Alkyl, $C_1$-$C_6$-Alkoxy oder Wasserstoff, mit der Massgabe, dass falls $R_4$ Wasserstoff darstellt, $R_1$, $R_2$ und $R_3$ nicht gleichzeitig Wasserstoff sind;
oder ferner das Brückenglied

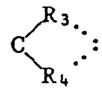 ,

welches zusammen auch einen gesättigten oder ungesättigten 3-7-gliedrigen Carbocyclus $W_1$ oder einen gesättigten oder ungesättigten 5-7-gliedrigen Heterocyclus $W_2$ mit 1 oder 2 Heteroatomen, die entweder Sauerstoff, Schwefel oder Stickstoff darstellen, bedeutet.

**3.** Verbindungen der Formel I gemäss Anspruch 1, worin bedeuten:

Y — Sauerstoff;
$Q_1$ und $Q_2$ — unabhängig voneinander Wasserstoff, Fluor, Chlor oder Brom;
$R_1$ und $R_2$ — unabhängig voneinander Wasserstoff, Methyl oder Ethyl;
$X_1$ — Wasserstoff, Halogen, $C_1$-$C_4$-Alkyl, $C_1$-$C_3$-Alkoxy, Phenyl, Phenoxy, durch Halogen oder $C_1$-$C_2$-Alkyl substituiertes Phenyl oder substituiertes Phenoxy;
$X_2$ — Wasserstoff oder Halogen;
$X_3$ — Wasserstoff;
A — die Brückenglieder

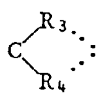 ,

Ethenylen, Ethinylen oder Carbonyl;
$R_3$ — $C_1$-$C_6$-Alkyl, $C_3$-$C_6$-Alkenyl, Hydroxy, $C_1$-$C_6$-Alkoxy, Cyano oder $COOC_1$-$C_3$-Alkyl;
$R_4$ — Wasserstoff, Methyl, Ethyl, Methoxy oder Ethoxy;
$W_1$ — Cycloheptyl, Cyclohexyl, Cyclopentyl, Cyclobutyl oder Cyclopropyl;
$W_2$ — 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

**4.** Verbindungen der Formel I gemäss Anspruch 1, worin bedeuten:

Y — Sauerstoff;
$Q_1$ und $Q_2$ — unabhängig voneinander Wasserstoff oder Fluor;
$R_1$ und $R_2$ — Wasserstoff oder Methyl;
$X_1$ — Wasserstoff, Halogen, Methyl, Ethyl, Methoxy, Ethoxy, Phenyl, Phenoxy, mit Fluor, Chlor, Methyl oder Ethyl substituiertes Phenyl oder substituiertes Phenoxy;
$X_2$ — Wasserstoff oder Halogen;
$X_3$ — Wasserstoff;
A — die Brückenglieder

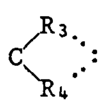 ,

Ethenylen oder Ethinylen;
$R_3$ — $C_2$-$C_4$-Alkyl, $C_3$-$C_6$-Alkenyl, Hydroxy, O-$C_1$-$C_4$-Alkyl, Cyano oder $COOC_1$-$C_3$-Alkyl;
$R_4$ — Wasserstoff, Methyl, Ethyl, Methoxy oder Ethoxy;
$W_1$ — Cyclopentyl oder Cyclohexyl;
$W_2$ — 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

**5.** Verbindungen der Formel I gemäss Anspruch 1, worin bedeuten:

Y Sauerstoff;
$Q_1$ und $Q_2$ Wasserstoff;
$R_1$ und $R_2$ Wasserstoff;
$X_1$ Wasserstoff, Fluor, Chlor, Methyl, Methoxy, Phenyl, Phenoxy, mit Fluor oder Methyl substituiertes Phenyl oder substituiertes Phenoxy;
$X_2$ Wasserstoff, Fluor oder Chlor;
$X_3$ Wasserstoff;
A die Brückenglieder

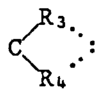 ,

Ethenylen oder Ethinylen;
$R_3$ Methyl, Ethyl, Allyl, Hydroxy, Methoxy oder Ethoxy;
$R_4$ Wasserstoff, Methyl, Ethyl, Methoxy oder Ethoxy;
$W_1$ Cyclopentyl oder Cyclohexyl;
$W_2$ 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

**6.** Verbindungen der Formel I gemäss Anspruch 1, worin bedeuten:

Y Sauerstoff;
$Q_1$ und $Q_2$ Wasserstoff;
$R_1$ und $R_2$ Wasserstoff;
$X_1$ Wasserstoff, Fluor, Chlor oder Methyl;
$X_2$ Wasserstoff, Fluor oder Chlor;
$X_3$ Wasserstoff;
A das Brückenglied

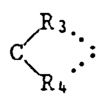 ;

$R_3$ Methyl, Ethyl, n-Propyl, i-Propyl, -n-Butyl, Allyl, Methoxy oder Ethoxy;
$R_4$ Wasserstoff, Methyl oder Ethyl.

**7.** Eine Verbindung der Formel I gemäss Anspruch 1 aus der Gruppe:

[Benzo-1,2,3-thiadiazol-7-carbonyloxymethyl]-2'-4'-dichlorphenylketon;
2-[Benzo-1,2,3-thiadiazol-7-carbonyloxymethyl]-1-phenyl-cyclopropan;
1-[Benzo-1,2,3-thiadiazol-7-carbonyloxymethyl]-1-(4-methoxyphenyl)-cyclopentan;
2-[Benzo-1,2,3-thiadiazol-7-carbonylthiomethyl]-1-phenyl-cyclopropan;
3-[Benzo-1,2,3-thiadiazol-7-carbonyloxymethyl]-1-phenyl-1-propen;
2-[Benzo-1,2,3-thiadiazol-7-carbonyloxy]-1-phenyl-n-propan;
2-[Benzo-1,2,3-thiadiazol-7-carbonyloxy]-1-phenyl-ethanol;
2-[Benzo-1,2,3-thiadiazol-7-carbonyloxy]-1-phenyl-propionsäuremethylester;
1-[Benzo-1,2,3-thiadiazol-7-carbonyloxy]-2-phenyl-n-propan;
1-[Benzo-1,2,3-thiadiazol-7-carbonyloxy]-2-phenyl-n-butan;
1-[Benzo-1,2,3-thiadiazol-7-carbonyloxy]-2-(2',4'-dichlorphenyl)-n-butan;
1-[6-Fluor-benzo-1,2,3-thiadiazol-7-carbonyloxy]-2-(2',4'-dichlorphenyl)-n-butan.

**8.** Verfahren zur Herstellung von Verbindungen der Formel I in Anspruch 1, dadurch gekennzeichnet, dass man umsetzt:

a) ein aktiviertes Carbonsäurederivat der Formel II

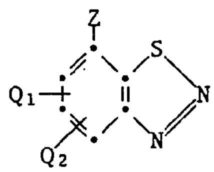

(II)

mit einem Alkohol oder Thiol der Formel III

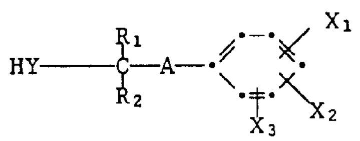

(III)

in Gegenwart einer Base gegebenenfalls mit einem Katalysator in inerten Lösungsmitteln bei Temperaturen von -20° bis 160°C, wobei Z die Reste Hal-Co,

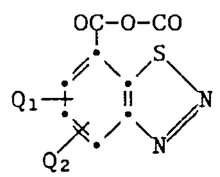

oder

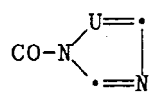

bedeutet, U, CH oder N und Hal Halogen darstellen; oder
b) die freie Säure der Formel II'

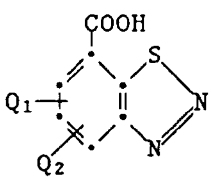

(II')

mit einem Alkohol oder Thiol der Formel III in Gegenwart eines sauren Katalysators in einem inerten Lösungsmittel oder im Ueberschuss der zur Veresterung eingesetzten Alkohol- oder Thiol-Komponente der Formel III bei Temperaturen von 10° bis 180°C, wobei die Reste oder Symbole A, $Q_1$, $Q_2$, $R_1$, $R_2$, $X_1$, $X_2$, $X_3$ und Y die unter Formel I angegebenen Bedeutungen besitzen.

**9.** Mittel zum Schutz von Pflanzen gegen den Befall durch Mikroorganismen, dadurch gekennzeichnet, dass es neben ublichen Trager- und Hilfsstoffen als aktive Komponente mindestens eine Verbindung gemäss Anspruch 1 enthalt.

**10.** Mittel gemäss Anspruch 9, dadurch gekennzeichnet, dass es als aktive Komponente mindestens eine Verbindung gemäss den Ansprüchen 2 bis 6 enthält.

**11.** Mittel gemäss Anspruch 9, dadurch gekennzeichnet, dass es als aktive Komponente die Verbindung der Formel I gemäss Anspruch 7 enthält.

**12.** Verfahren zur Herstellung eines agrochemischen Mittels von Anspruch 9, dadurch gekennzeichnet, dass man mindestens eine gemäss Anspruch 1 definierte Verbindung mit geeigneten festen oder flüssigen Träger- und Hilfsstoffen innig vermischt.

**13.** Verwendung von Verbindungen gemäss Anspruch 1 zum Schutz von Pflanzen gegen den Befall durch phytopathogene Mikroorganismen.

**14.** Verwendung von Verbindungen gemäss einem der Ansprüche 2 bis 7 zum Schutz von Pflanzen gegen den Befall durch phytopathogene Mikroorganismen.

15. Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

16. Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 7 auf die Pflanze oder deren Standort appliziert.

17. Verfahren gemäss den Ansprüchen 15 und 16, dadurch gekennzeichnet, dass es sich bei den phytopathogenen Mikroorganismen um Pilzorganismen handelt.

**Patentansprüche für folgenden Vertragsstaat : ES**

1. Verfahren zur Herstellung von Verbindungen der Formel I

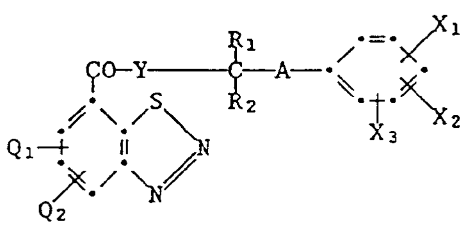

(I)

in welcher bedeuten:

| | |
|---|---|
| Y | Sauerstoff oder Schwefel; |
| $Q_1$ und $Q_2$ | unabhängig voneinander Wasserstoff oder Halogen; |
| $R_1$ und $R_2$ | unabhängig voneinander Wasserstoff oder $C_1$-$C_4$-Alkyl; |
| $X_1$ | Wasserstoff, Halogen, $C_1$-$C_4$-Alkyl, Trifluormethyl, $C_1$-$C_3$-Alkyl, im Alkylteil durch Halogen substituiertes $C_1$-$C_2$-Alkoxy, Nitro, Cyano, Dimethylamino, Phenyl, Phenoxy, durch Halogen und/oder $C_1$-$C_2$-Alkyl und/oder $C_1$-$C_2$-Alkoxy substituiertes Phenyl oder Phenoxy; |
| $X_2$ | Wasserstoff, Halogen oder Methyl; |
| $X_3$ | Wasserstoff oder Halogen; |
| | und |
| A | die Brückenglieder |

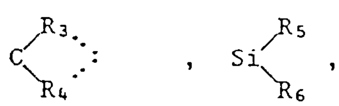

Carbonyl, Ethenylen oder Ethinylen umfasst;
wobei

| | |
|---|---|
| $R_3$ | $C_1$-$C_6$-Alkyl, $C_3$-$C_6$-Alkenyl, Hydroxy, $C_1$-$C_6$-Alkoxy, O-C(O)-$C_1$-$C_4$-Alkyl, Carboxyl, $COOC_1$-$C_4$-Alkyl, Cyano oder Wasserstoff, mit der Massgabe, dass falls $R_3$ Wasserstoff darstellt, $R_1$, $R_2$ und $R_4$ nicht gleichzeitig Wasserstoff sind; |
| $R_4$ | $C_1$-$C_6$-Alkyl, $C_1$-$C_6$-Alkoxy oder Wasserstoff, mit der Massgabe, dass falls $R_4$ Wasserstoff darstellt, $R_1$, $R_2$ und $R_3$ nicht gleichzeitig Wasserstoff sind; |
| $R_5$ | $C_3$-$C_6$-Alkyl, $C_3$-$C_6$-Alkyl,$C_1$-$C_6$-Alkoxy, Phenyl, oder durch Halogen oder Methoxy substituiertes Phenyl; |
| $R_6$ | $C_1$-$C_6$-Alkyl oder $C_1$-$C_6$-Alkoxy; |
| | oder ferner das Brückenglied |

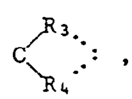

welches zusammen auch einen gesättigten oder ungesättigten 3-7-gliedrigen Carbocyc-

lus $W_1$ oder einen gesättigten oder ungesättigten 5-7-gliedrigen Heterocyclus $W_2$ mit 1 oder 2 Heteroatomen, die entweder Sauerstoff, Schwefel oder Stickstoff darstellen, bedeutet;

oder das Brückenglied

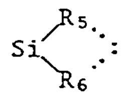 ,

welches zusammen einen gesättigten oder ungesättigten 3 bis 7-gliedrigen Heterocyclus $W_3$ mit maximal 2 Sauerstoff- oder Schwefelatomen als weitere Heteroatome bedeutet, dadurch gekennzeichnet, dass man umsetzt:

a) ein aktiviertes Carbonsäurederivat der Formel II

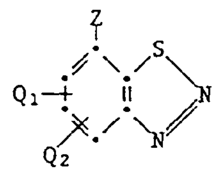

(II)

mit einem Alkohol oder Thiol der Formel III

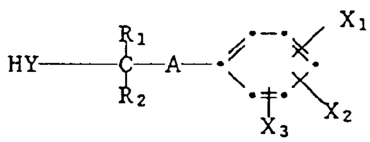

(III)

in Gegenwart einer Base gegebenenfalls mit einem Katalysator in inerten Lösungsmitteln bei Temperaturen von -20° bis 160°C, wobei Z die Reste Hal-Co,

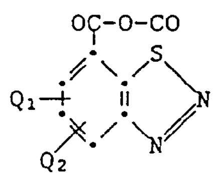

oder

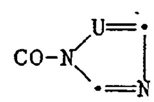

bedeutet, U, CH oder N und Hal Halogen darstellen; oder

b) die freie Saure der Formel II'

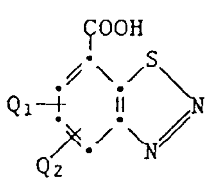

(II')

mit einem Alkohol oder Thiol der Formel III in Gegenwart eines sauren Katalysators in einem inerten Losungsmittel oder im Ueberschuss der zur Veresterung eingesetzten Alkohol- oder Thiol-Komponente der Formel III bei Temperaturen von 10° bis 180°C, wobei die Reste oder Symbole A, $Q_1$. $Q_2$, $R_1$, $R_2$, $X_1$, $X_2$, $X_3$ und Y die unter Formel I angegebenen Bedeutungen besitzen.

**2.** Verfahren zur Herstellung von Verbindungen der Formel I gemäss Anspruch 1, worin bedeuten:

Y Sauerstoff oder Schwefel;

$Q_1$ und $Q_2$ unabhängig voneinander Wasserstoff oder Halogen;

$R_1$ und $R_2$ unabhängig voneinander Wasserstoff oder $C_1$-$C_4$-Alkyl;

$X_1$ Wasserstoff, Halogen, $C_1$-$C_4$-Alkyl, Trifluormethyl, $C_1$-$C_3$-Alkyl, im Alkylteil durch Halogen substituiertes $C_1$-$C_2$-Alkoxy, Nitro, Cyano, Dimethylamino, Phenyl, Phenoxy, durch Halogen und/oder $C_1$-$C_2$-Alkyl und/oder $C_1$-$C_2$-Alkoxy substituiertes Phenyl oder Phenoxy;

$X_2$ Wasserstoff, Halogen oder Methyl;

$X_3$ Wasserstoff oder Halogen;

und

A die Brückenglieder

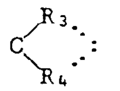 ,

Carbonyl, Ethenylen oder Ethinylen umfasst;

wobei

$R_3$ $C_1$-$C_6$-Alkyl, $C_3$-$C_6$-Alkenyl, Hydroxy, $C_1$-$C_6$-Alkoxy, O-C(O)-$C_1$-$C_4$-Alkyl, Carboxyl, $COOC_1$-$C_4$-Alkyl, Cyano oder Wasserstoff, mit der Massgabe, dass falls $R_3$ Wassertoff darstellt, $R_1$, $R_2$ und $R_4$ nicht gleichzeitig Wasserstoff sind;

$R_4$ $C_1$-$C_6$-Alkyl, $C_1$-$C_6$-Alkoxy oder Wasserstoff, mit der Massgabe, dass falls $R_4$ Wasserstoff darstellt, $R_1$, $R_2$ und $R_3$ nicht gleichzeitig Wasserstoff sind;

oder ferner das Brückenglied

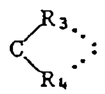 ,

welches zusammen auch einen gesättigten oder ungesättigten 3-7-gliedrigen Carbocyclus $W_1$ oder einen gesättigten oder ungesättigten 5-7-gliedrigen Heterocyclus $W_2$ mit 1 oder 2 Heteroatomen, die entweder Sauerstoff, Schwefel oder Stickstoff darstellen, bedeutet.

3. Verfahren zur Herstellung von Verbindungen der Formel I gemäss Anspruch 1, worin bedeuten:

Y Sauerstoff;

$Q_1$ und $Q_2$ unabhängig voneinander Wasserstoff, Fluor, Chlor oder Brom;

$R_1$ und $R_2$ unabhängig voneinander Wasserstoff, Methyl oder Ethyl;

$X_1$ Wasserstoff, Halogen, $C_1$-$C_4$-Alkyl, $C_1$-$C_3$-Alkoxy, Phenyl, Phenoxy, durch Halogen oder $C_1$-$C_2$-Alkyl substituiertes Phenyl oder substituiertes Phenoxy;

$X_2$ Wasserstoff oder Halogen;

$X_3$ Wasserstoff;

A die Brückenglieder

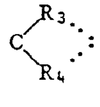 ,

Ethenylen, Ethinylen oder Carbonyl;

$R_3$ $C_1$-$C_6$-Alkyl, $C_3$-$C_6$-Alkenyl, Hydroxy, $C_1$-$C_6$-Alkoxy, Cyano oder $COOC_1$-$C_3$-Alkyl;

$R_4$ Wasserstoff, Methyl, Ethyl, Methoxy oder Ethoxy;

$W_1$ Cycloheptyl, Cyclohexyl, Cyclopentyl, Cyclobutyl oder Cyclopropyl;

$W_2$ 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

4. Verfahren zur Herstellung von Verbindungen der Formel I gemäss Anspruch 1, worin bedeuten:

Y Sauerstoff;

$Q_1$ und $Q_2$ unabhängig voneinander Wasserstoff oder Fluor;

$R_1$ und $R_2$ Wasserstoff oder Methyl;
$X_1$ Wasserstoff, Halogen, Methyl, Ethyl, Methoxy, Ethoxy, Phenyl, Phenoxy, mit Fluor, Chlor, Methyl oder Ethyl substituiertes Phenyl oder substituiertes Phenoxy;
$X_2$ Wasserstoff oder Halogen;
$X_3$ Wasserstoff;
A die Brückenglieder

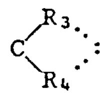 ,

Ethenylen oder Ethinylen;
$R_3$ $C_2$-$C_4$-Alkyl, $C_3$-$C_6$-Alkenyl, Hydroxy, O-$C_1$-$C_4$-Alkyl, Cyano oder COO$C_1$-$C_3$-Alkyl;
$R_4$ Wasserstoff, Methyl, Ethyl, Methoxy oder Ethoxy;
$W_1$ Cyclopentyl oder Cyclohexyl;
$W_2$ 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

**5.** Verfahren zur Herstellung von Verbindungen der Formel I gemäss Anspruch 1, worin bedeuten:
Y Sauerstoff;
$Q_1$ und $Q_2$ Wasserstoff;
$R_1$ und $R_2$ Wasserstoff;
$X_1$ Wasserstoff, Fluor, Chlor, Methyl, Methoxy, Phenyl, Phenoxy, mit Fluor oder Methyl substituiertes Phenyl oder substituiertes Phenoxy;
$X_2$ Wasserstoff, Fluor oder Chlor;
$X_3$ Wasserstoff;
A die Brückenglieder

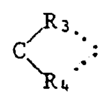 ,

Ethenylen oder Ethinylen;
$R_3$ Methyl, Ethyl, Allyl, Hydroxy, Methoxy oder Ethoxy;
$R_4$ Wasserstoff, Methyl, Ethyl, Methoxy oder Ethoxy;
$W_1$ Cyclopentyl oder Cyclohexyl;
$W_2$ 1,3-Dioxolan-2-yl, 1,3-Dioxan-2-yl, 1,3-Dioxolan-4-on-5-yl, Tetrahydrofuran-2-on-5-yl, 1,3-Dithian-2-yl, Tetrahydro-1,3-oxazin-2-yl oder Oxazolidin-2-yl.

**6.** Verfahren zur Herstellung von Verbindungen der Formel I gemäss Anspruch 1, worin bedeuten:
Y Sauerstoff;
$Q_1$ und $Q_2$ Wasserstoff;
$R_1$ und $R_2$ Wasserstoff;
$X_1$ Wasserstoff, Fluor, Chlor oder Methyl;
$X_2$ Wasserstoff, Fluor oder Chlor;
$X_3$ Wasserstoff;
A das Brückenglied

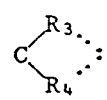 ;

$R_3$ Methyl, Ethyl, n-Propyl, i-Propyl, -n-Butyl, Allyl, Methoxy oder Ethoxy;
$R_4$ Wasserstoff, Methyl oder Ethyl.

**7.** Verfahren zur Herstellung einer Verbindungen der Formel I gemäss Anspruch 1 aus der Gruppe:

[Benzo-1,2,3-thiadiazol-7-carbonyloxymethyl]-2'-4'-dichlorphenylketon;
2-[Benzo-1,2,3-thiadiazol-7-carbonyloxymethyl]-1-phenyl-cyclopropan;
1-[Benzo-1,2,3-thiadiazol-7-carbonyloxymethyl]-1-(4-methoxyphenyl)-cyclopentan;
2-[Benzo-1,2,3-thiadiazol-7-carbonylthiomethyl]-1-phenyl-cyclopropan;
3-[Benzo-1,2,3-thiadiazol-7-carbonyloxymethyl]-1-phenyl-1-propen;
2-[Benzo-1,2,3-thiadiazol-7-carbonyloxy]-1-phenyl-n-propan;
2-[Benzo-1,2,3-thiadiazol-7-carbonyloxy]-1-phenyl-ethanol;
2-[Benzo-1,2,3-thiadiazol-7-carbonyloxy]-1-phenyl-propionsäuremethylester;
1-[Benzo-1,2,3-thiadiazol-7-carbonyloxy]-2-phenyl-n-propan;
1-[Benzo-1,2,3-thiadiazol-7-carbonyloxy]-2-phenyl-n-butan;
1-[Benzo-1,2,3-thiadiazol-7-carbonyloxy]-2-(2',4'-dichlorphenyl)-n-butan;
1-[6-Fluor-benzo-1,2,3-thiadiazol-7-carbonyloxy]-2-(2',4'-dichlorphenyl)-n-butan.

**8.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

**9.** Mittel gemäss Anspruch 8, dadurch gekennzeichnet, dass es als aktive Komponente mindestens eine Verbindung gemäss den Ansprüchen 2 bis 6 enthalt.

**10.** Mittel gemäss Anspruch 8, dadurch gekennzeichnet, dass es als aktive Komponente die Verbindung der Formel I gemäss Anspruch 7 enthält.

**11.** Verfahren zur Herstellung eines agrochemischen Mittels von Anspruch 8, dadurch gekennzeichnet, dass man mindestens eine gemäss Anspruch 1 definierte Verbindung mit geeigneten festen oder flüssigen Träger- und Hilfsstoffen innig vermischt.

**12.** Verwendung von Verbindungen gemäss Anspruch 1 zum Schutz von Pflanzen gegen den Befall durch phytopathogene Mikroorganismen.

**13.** Verwendung von Verbindungen gemäss einem der Ansprüche 2 bis 7 zum Schutz von Pflanzen gegen den Befall durch phytopathogene Mikroorganismen.

**14.** Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

**15.** Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 7 auf die Pflanze oder deren Standort appliziert.

**16.** Verfahren gemäss den Ansprüchen 14 und 15, dadurch gekennzeichnet, dass es sich bei den phytopathogenen Mikroorganismen um Pilzorganismen handelt.

## Claims

### Claims for the following Contracting States : AT, BE, CH, DE, DK, FR, GB, GR, IT, LI, LU, NL, SE

**1.** A compound of formula I

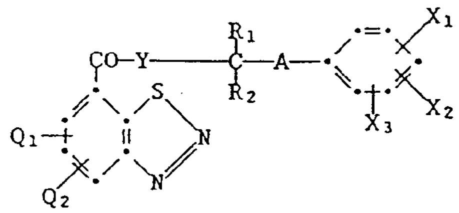

(I)

in which
Y is oxygen or sulfur;
$Q_1$ and $Q_2$ independently of one another are each hydrogen or halogen;
$R_1$ and $R_2$ independently of one another are each hydrogen or $C_1$-$C_4$alkyl;
$X_1$ is hydrogen, halogen, $C_1$-$C_4$alkyl, trifluoromethyl, $C_1$-$C_3$alkyl, $C_1$-$C_2$alkoxy halo-substituted in the alkyl moiety, nitro, cyano, dimethylamino, phenyl, phenoxy, or phenyl or phenoxy each substituted by halogen and/or by $C_1$-$C_2$alkyl and/or by $C_1$-$C_2$alkoxy;
$X_2$ is hydrogen, halogen or methyl;
$X_3$ is hydrogen or halogen; and
A Comprises the bridge members

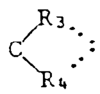 , 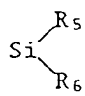 ,

carbonyl, ethenylene and ethynylene, wherein
$R_3$ is $C_1$-$C_6$alkyl, $C_3$-$C_6$alkenyl, hydroxy, $C_1$-$C_6$alkoxy, O-C(O)-$C_1$-$C_4$alkyl, carboxy, COO$C_1$-$C_4$alkyl, cyano or hydrogen, with the proviso that if $R_3$ is hydrogen $R_1$, $R_2$ and $R_4$ are not simultaneously hydrogen;
$R_4$ is $C_1$-$C_6$alkyl, $C_1$-$C_6$alkoxy or hydrogen, with the proviso that if $R_4$ is hydrogen $R_1$, $R_2$ and $R_3$ are not simultaneously hydrogen;
$R_5$ is $C_1$-$C_6$alkyl, $C_3$-$C_6$cycloalkyl, $C_1$-$C_6$alkoxy, phenyl, or phenyl substituted by halogen or by methoxy;
$R_6$ is $C_1$-$C_6$alkyl or $C_1$-$C_6$alkoxy;
or alternatively A represents the bridge member

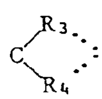

that together also represents a saturated or unsaturated 3- to 7-membered carbocycle $W_1$ or a saturated or unsaturated 5- to 7-membered heterocycle $W_2$ having 1 or 2 hetero atoms that are either oxygen, sulfur or nitrogen; or the bridge member

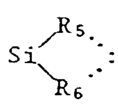

that together represents a saturated or unsaturated 3- to 7-membered heterocycle $W_3$ having a maximum of 2 oxygen or sulfur atoms as additional hetero atoms.

**2.** A compound of formula I according to claim 1, in which
Y is oxygen or sulfur;
$Q_1$ and $Q_2$ independently of one another are each hydrogen or halogen;
$R_1$ and $R_2$ independently of one another are each hydrogen or $C_1$-$C_4$alkyl;
$X_1$ is hydrogen, halogen, $C_1$-$C_4$alkyl, trifluoromethyl, $C_1$-$C_3$alkyl, $C_1$-$C_2$alkoxy halo-substituted in the alkyl moiety, nitro, cyano, dimethylamino, phenyl, phenoxy, or phenyl or phenoxy each substituted by halogen and/or by $C_1$-$C_2$alkyl and/or by $C_1$-$C_2$alkoxy;
$X_2$ is hydrogen, halogen or methyl;

$X_3$ is hydrogen or halogen; and
A comprises the bridge members

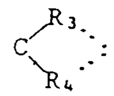
,

carbonyl, ethenylene and ethynylene wherein
$R_3$ is $C_1$-$C_6$alkyl, $C_3$-$C_6$alkenyl, hydroxy, C1-C6alkoxy, O-C(O)-$C_1$-$C_4$alkyl, carboxy, COOC$_1$-$C_4$alkyl, cyano or hydrogen, with the proviso that if $R_3$ is hydrogen $R_1$, $R_2$ and $R_4$ are not simultaneously hydrogen;
$R_4$ is $C_1$-$C_6$alkyl, $C_1$-$C_6$alkoxy or hydrogen, with the proviso that if $R_4$ is hydrogen $R_1$, $R_2$ and $R_3$ are not simultaneously hydrogen; or alternatively A represents the bridge member

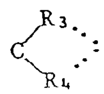

that together also represents a saturated or unsaturated 3- to 7-membered carbocycle $W_1$ or a saturated or unsaturated 5- to 7-membered heterocycle $W_2$ having 1 or 2 hetero atoms that are either oxygen, sulfur or nitrogen.

**3.** A compound of formula I according to claim 1, in which
Y is oxygen;
$Q_1$ and $Q_2$ independently of one another are each hydrogen, fluorine, chlorine or bromine;
$R_1$ and $R_2$ independently of one another are each hydrogen, methyl or ethyl;
$X_1$ is hydrogen, halogen, $C_1$-$C_4$alkyl, $C_1$-$C_3$alkoxy, phenyl, phenoxy, or phenyl or phenoxy each substituted by halogen or by $C_1$-$C_2$alkyl;
$X_2$ is hydrogen or halogen;
$X_3$ is hydrogen;
A represents the bridge members

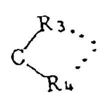
,

ethenylene, ethynylene and carbonyl;
$R_3$ is $C_1$-$C_6$alkyl, $C_3$-$C_6$alkenyl, hydroxy, $C_1$-$C_6$alkoxy, cyano or COOC$_1$-$C_3$alkyl;
$R_4$ is hydrogen, methyl, ethyl, methoxy or ethoxy;
$W_1$ is cycloheptyl, cyclohexyl, cyclopentyl, cyclobutyl or cyclopropyl;
$W_2$ is 1,3-dioxolan-2-yl, 1,3-dioxan-2-yl, 1,3-dioxolan-4-on-5-yl, tetrahydrofuran-2-on-5-yl, 1,3-dithian-2-yl, tetrahydro-1,3-oxazin-2-yl or oxazolidin-2-yl.

**4.** A compound of formula I according to claim 1, in which
Y is oxygen;
$Q_1$ and $Q_2$ independently of one another are each hydrogen or fluorine;
$R_1$ and $R_2$ are hydrogen or methyl;
$X_1$ is hydrogen, halogen, methyl, ethyl, methoxy, ethoxy, phenyl, phenoxy, or phenyl or phenoxy each substituted by fluorine, chlorine, methyl or by ethyl;
$X_2$ is hydrogen or halogen;
$X_3$ is hydrogen;
A represents the bridge members

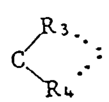
,

ethenylene and ethynylene;
$R_3$ is $C_2$-$C_4$alkyl, $C_3$-$C_6$alkenyl, hydroxy, O-$C_1$-$C_4$alkyl, cyano or $COOC_1$-$C_3$alkyl;
$R_4$ is hydrogen, methyl, ethyl, methoxy or ethoxy;
$W_1$ is cyclopentyl or cyclohexyl;
$W_2$ is 1,3-dioxolan-2-yl, 1,3-dioxan-2-yl, 1,3-dioxolan-4-on-5-yl, tetrahydrofuran-2-on-5-yl, 1,3-dithian-2-yl, tetrahydro-1,3-oxazin-2-yl or oxazolidin-2-yl.

5. A compound of formula I according to claim 1, in which
Y is oxygen;
$Q_1$ and $Q_2$ are hydrogen;
$R_1$ and $R_2$ are hydrogen;
$X_1$ is hydrogen, fluorine, chlorine, methyl, methoxy, phenyl, phenoxy, or phenyl or phenoxy each substituted by fluorine or by methyl;
$X_2$ is hydrogen, fluorine or chlorine;
$X_3$ is hydrogen;
A represents the bridge members

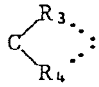 ,

ethenylene and ethynylene;
$R_3$ is methyl, ethyl, allyl, hydroxy, methoxy or ethoxy;
$R_4$ is hydrogen, methyl, ethyl, methoxy or ethoxy;
$W_1$ is cyclopentyl or cyclohexyl;
$W_2$ is 1,3-dioxolan-2-yl, 1,3-dioxan-2-yl, 1,3-dioxolan-4-on-5-yl, tetrahydrofuran-2-on-5-yl, 1,3-dithian-2-yl, tetrahydro-1,3-oxazin-2-yl or oxazolidin-2-yl.

6. A compound of formula I according to claim 1, in which
Y is oxygen;
$Q_1$ and $Q_2$ are hydrogen;
$R_1$ and $R_2$ are hydrogen;
$X_1$ is hydrogen, fluorine, chlorine or methyl;
$X_2$ is hydrogen, fluorine or chlorine;
$X_3$ is hydrogen;
A represents the bridge member

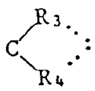 ;

$R_3$ is methyl, ethyl, n-propyl, i-propyl, n-butyl, allyl, methoxy or ethoxy;
$R_4$ is hydrogen, methyl or ethyl.

7. A compound of formula I according to claim 1 from the group:
[benzo-1,2,3-thiadiazole-7-carbonyloxymethyl]-2',4'-dichlorophenylketone;
2-[benzo-1,2,3-thiadiazole-7-carbonyloxymethyl]-1-phenylcyclopropane;
1-[benzo-1,2,3-thiadiazole-7-carbonyloxymethyl]-1-(4-methoxyphenyl)-cyclopentane;
2-[benzo-1,2,3-thiadiazole-7-carbonylthiomethyl]-1-phenylcyclopropane;
3-[benzo-1,2,3-thiadiazole-7-carbonyloxymethyl]-1-phenyl-1-propene;
2- [benzo-1,2,3-thiadiazole-7-carbonyloxy]-1-phenyl-n-propane;
2-[benzo-1,2,3-thiadiazole-7-carbonyloxy]-1-phenyl-ethanol;
2-[benzo-1,2,3-thiadiazole-7-carbonyloxy]-1-phenyl-propionic acid methyl ester;
1-[benzo-1,2,3-thiadiazole-7-carbonyloxy]-2-phenyl-n-propane;
1-[benzo-1,2,3-thiadiazole-7-carbonyloxy]-2-phenyl-n-butane;
1-[benzo-1,2,3-thiadiazole-7-carbonyloxy]-2-(2',4'-dichlorophenyl)-n-butane;
1-[6-fluorobenzo-1,2,3-thiadiazole-7-carbonyloxy]-2-(2',4,-dichlorophenyl)-n-butane.

**8.** A process for the preparation of a compound of formula I as claimed in claim 1 which comprises reacting:
a) an activated carboxylic acid derivative of formula II

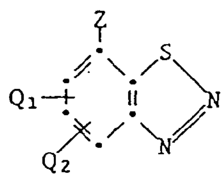

(II)

with an alcohol or thiol of formula III

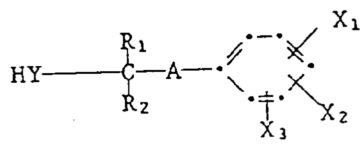

(III)

in the presence of a base and optionally with a catalyst, in an inert solvent, at temperatures of from -20° to 160°C, in which formulae Z represents the radicals Hal-CO,

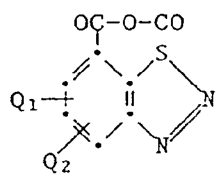

and

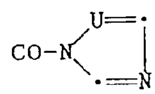

,

U is CH or N and Hal is halogen; or
b) the free acid of formula II'

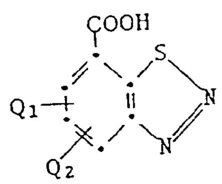

(II')

with an alcohol or thiol of formula III in the presence of an acidic catalyst, in an inert solvent, or in an excess of the alcohol or thiol component of formula III used for the esterification, at temperatures of from 10 to 180°C, the radicals or symbols A, $Q_1$, $Q_2$, $R_1$, $R_2$, $X_1$, $X_2$, $X_3$ and Y being as defined for formula I.

**9.** A composition for protecting plants against attack by microorganisms that comprises as active component at least one compound according to claim 1 together with customary carriers and adjuvants.

**10.** A composition according to claim 9 that comprises as active component at least one compound according to claims 2 to 6.

**11.** A composition according to claim 9 that comprises as active component a compound of formula I according to claim 7.

**12.** A process for the preparation of an agrochemical composition as claimed in claim 9 which comprises homogeneously mixing at least one compound defined in accordance with claim 1 with suitable solid or liquid carriers and adjuvants.

**13.** The use of a compound according to claim 1 for protecting plants against attack by phytopathogenic microorganisms.

14. The use of a compound according to any one of claims 2 to 7 for protecting plants against attack by phytopathogenic microorganisms.

15. A method of protecting plants against attack by phytopathogenic microorganisms which comprises applying as active ingredient to the plant or the locus thereof a compound according to claim 1.

16. A method of protecting plants against attack by phytopathogenic microorganisms which comprises applying as active ingredient to the plant or the locus thereof a compound according to any one of claims 2 to 7.

17. A method according to claims 15 and 16 wherein the phytopathogenic microorganisms are fungal organisms.

**Claims for the following Contracting State : ES**

1. A process for the preparation of a compound of formula I

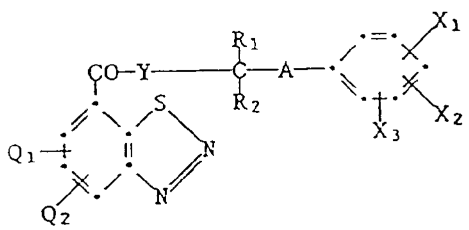

(I)

in which
Y is oxygen or sulfur;
$Q_1$ and $Q_2$ independently of one another are each hydrogen or halogen;
$R_1$ and $R_2$ independently of one another are each hydrogen or $C_1$-$C_4$alkyl;
$X_1$ is hydrogen, halogen, $C_1$-$C_4$alkyl, trifluoromethyl, $C_1$-$C_3$alkyl, $C_1$-$C_2$alkoxy halo-substituted in the alkyl moiety, nitro, cyano, dimethylamino, phenyl, phenoxy, or phenyl or phenoxy each substituted by halogen and/or by $C_1$-$C_2$alkyl and/or by $C_1$-$C_2$alkoxy;
$X_2$ is hydrogen, halogen or methyl;
$X_3$ is hydrogen or halogen; and
A comprises the bridge members

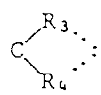 , 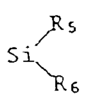 ,

carbonyl, ethenylene and ethynylene, wherein
$R_3$ is $C_1$-$C_6$alkyl, $C_3$-$C_6$alkenyl, hydroxy, $C_1$-$C_6$alkoxy, O-C(O)-$C_1$-$C_4$alkyl, carboxy, COO$C_1$-$C_4$alkyl, cyano or hydrogen, with the proviso that if $R_3$ is hydrogen $R_1$, $R_2$ and $R_4$ are not simultaneously hydrogen;
$R_4$ is $C_1$-$C_6$alkyl, $C_1$-$C_6$alkoxy or hydrogen, with the proviso that if $R_4$ is hydrogen $R_1$, $R_2$ and $R_3$ are not simultaneously hydrogen;
$R_5$ is $C_1$-$C_6$alkyl, $C_3$-$C_6$cycloalkyl, $C_1$-$C_6$alkoxy, phenyl, or phenyl substituted by halogen or by methoxy;
$R_6$ is $C_1$-$C_6$alkyl or $C_1$-$C_6$alkoxy;
or alternatively A represents the bridge member

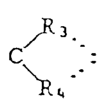

that together also represents a saturated or unsaturated 3- to 7-membered carbocycle $W_1$ or a saturated or unsaturated 5- to 7-membered heterocycle $W_2$ having 1 or 2 hetero atoms that are either oxygen, sulfur or nitrogen; or the bridge member

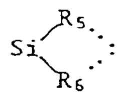

that together represents a saturated or unsaturated 3- to 7-membered heterocycle $W_3$ having a maximum of 2 oxygen or sulfur atoms as additional hetero atoms, which comprises reacting

a) an activated carboxylic acid derivative of formula II

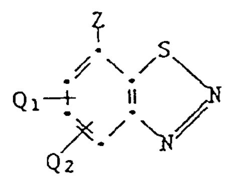

(II)

with an alcohol or thiol of formula III

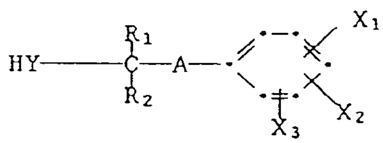

(III)

in the presence of a base and optionally with a catalyst, in an inert solvent, at temperatures of from -20 to 160°C, in which formulae Z represents the radicals Hal-CO,

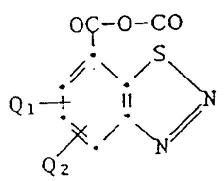

and

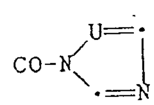

,

U is CH or N and Hal is halogen; or

b) the free acid of formula II'

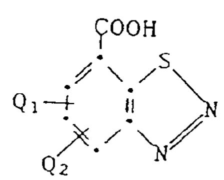

(II')

with an alcohol or thiol of formula III in the presence of an acidic catalyst, in an inert solvent, or in an excess of the alcohol or thiol component of formula III used for the esterification, at temperatures of from 10 to 180°C, the radicals or symbols A, $Q_1$, $Q_2$, $R_1$, $R_2$, $X_1$, $X_2$, $X_3$ and Y being as defined for formula I.

**2.** A process for the preparation of a compound of formula I according to claim 1, in which
Y is oxygen or sulfur;
$Q_1$ and $Q_2$ independently of one another are each hydrogen or halogen;
$R_1$ and $R_2$ independently of one another are each hydrogen or $C_1$-$C_4$alkyl;
$X_1$ is hydrogen, halogen, $C_1$-$C_4$alkyl, trifluoromethyl, $C_1$-$C_3$alkyl, $C_1$-$C_2$alkoxy halo-substituted in the alkyl moiety, nitro, cyano, dimethylamino, phenyl, phenoxy, or phenyl or phenoxy each substituted by halogen and/or by $C_1$-$C_2$alkyl and/or by $C_1$-$C_2$alkoxy;
$X_2$ is hydrogen, halogen or methyl;
$X_3$ is hydrogen or halogen; and
A comprises the bridge members

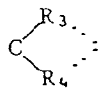 ,

carbonyl, ethenylene and ethynylene wherein
$R_3$ is $C_1$-$C_6$alkyl, $C_3$-$C_6$alkenyl, hydroxy, $C_1$-$C_6$alkoxy, O-C(O)-$C_1$-$C_4$alkyl, carboxy, $COOC_1$-$C_4$alkyl, cyano or hydrogen, with the proviso that if $R_3$ is hydrogen $R_1$, $R_2$ and $R_4$ are not simultaneously hydrogen;
$R_4$ is $C_1$-$C_6$alkyl, $C_1$-$C_6$alkoxy or hydrogen, with the proviso that if $R_4$ is hydrogen $R_1$, $R_2$ and $R_3$ are not simultaneously hydrogen;
or alternatively A represents the bridge member

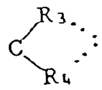

that together also represents a saturated or unsaturated 3- to 7-membered carbocycle $W_1$ or a saturated or unsaturated 5- to 7-membered heterocycle $W_2$ having 1 or 2 hetero atoms that are either oxygen, sulfur or nitrogen.

**3.** A process for the preparation of a compound of formula I according to claim 1, in which
Y is oxygen;
$Q_1$ and $Q_2$ independently of one another are each hydrogen, fluorine, chlorine or bromine;
$R_1$ and $R_2$ independently of one another are each hydrogen, methyl or ethyl;
$X_1$ is hydrogen, halogen, $C_1$-$C_4$alkyl, $C_1$-$C_3$alkoxy, phenyl, phenoxy, or phenyl or phenoxy each substituted by halogen or by $C_1$-$C_2$alkyl;
$X_2$ is hydrogen or halogen;
$X_3$ hydrogen;
A represents the bridge members

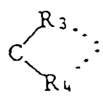 ,

ethenylene, ethynylene and carbonyl;
$R_3$ is $C_1$-$C_6$alkyl, $C_3$-$C_6$alkenyl, hydroxy, $C_1$-$C_6$alkoxy, cyano or $COOC_1$-$C_3$alkyl;
$R_4$ is hydrogen, methyl, ethyl, methoxy or ethoxy;
$W_1$ is cycloheptyl, cyclohexyl, cyclopentyl, cyclobutyl or cyclopropyl;
$W_2$ is 1,3-dioxolan-2-yl, 1,3-dioxan-2-yl, 1,3-dioxolan-4-on-5-yl, tetrahydrofuran-2-on-5-yl, 1,3-dithian-2-yl, tetrahydro-1,3-oxazin-2-yl or oxazolidin-2-yl.

**4.** A process for the preparation of a compound of formula I according to claim 1, in which
Y is oxygen;
$Q_1$ and $Q_2$ independently of one another are each hydrogen or fluorine;
$R_1$ and $R_2$ are hydrogen or methyl;
$X_1$ is hydrogen, halogen, methyl, ethyl, methoxy, ethoxy, phenyl, phenoxy, or phenyl or phenoxy each substituted by fluorine, chlorine, methyl or by ethyl;
$X_2$ is hydrogen or halogen;
$X_3$ is hydrogen;
A represents the bridge members

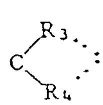 ,

ethenylene and ethynylene;

$R_3$ is $C_2$-$C_4$alkyl, $C_3$-$C_6$alkenyl, hydroxy, O-$C_1$-$C_4$alkyl, cyano or $COOC_1$-$C_3$alkyl;
$R_4$ is hydrogen, methyl, ethyl, methoxy or ethoxy;
$W_1$ is cyclopentyl or cyclohexyl;
$W_2$ is 1,3-dioxolan-2-yl, 1,3-dioxan-2-yl, 1,3-dioxolan-4-on-5-yl, tetrahydrofuran-2-on-5-yl, 1,3-dithian-2-yl, tetrahydro-1,3-oxazin-2-yl or oxazolidin-2-yl.

5. A process for the preparation of a compound of formula I according to claim 1, in which
Y is oxygen;
$Q_1$ and $Q_2$ are hydrogen;
$R_1$ and $R_2$ are hydrogen;
$X_1$ is hydrogen, fluorine, chlorine, methyl, methoxy, phenyl, phenoxy, or phenyl or phenoxy each substituted by fluorine or by methyl;
$X_2$ is hydrogen, fluorine or chlorine;
$X_3$ is hydrogen;
A represents the bridge members

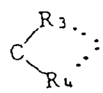 ,

ethenylene and ethynylene;
$R_3$ is methyl, ethyl, allyl, hydroxy, methoxy or ethoxy;
$R_4$ is hydrogen, methyl, ethyl, methoxy or ethoxy;
$W_1$ is cyclopentyl or cyclohexyl;
$W_2$ is 1,3-dioxolan-2-yl, 1,3-dioxan-2-yl, 1,3-dioxolan-4-on-5-yl, tetrahydrofuran-2-on-5-yl, 1,3-dithian-2-yl, tetrahydro-1,3-oxazin-2-yl or oxazolidin-2-yl.

6. A process for the preparation of a compound of formula I according to claim 1, in which
Y is oxygen;
$Q_1$ and $Q_2$ are hydrogen;
$R_1$ and $R_2$ are hydrogen;
$X_1$ is hydrogen, fluorine, chlorine or methyl;
$X_2$ is hydrogen, fluorine or chlorine;
$X_3$ is hydrogen;
A represents the bridge member

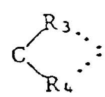 ;

$R_3$ is methyl, ethyl, n-propyl, i-propyl, n-butyl, allyl, methoxy or ethoxy;
$R_4$ is hydrogen, methyl or ethyl.

7. A process for the preparation of a compound of formula I according to claim 1 from the group:
[benzo-1,2,3-thiadiazole-7-carbonyloxymethyl]-2',4,'-dichlorophenylketone;
2-[benzo-1,2,3-thiadiazole-7-carbonyloxymethyl]-1-phenylcyclopropane;
1-[benzo-1,2,3-thiadiazole-7-carbonyloxymethyl]-1(4-methoxyphenyl)-cyclopentane;
2-[benzo-1,2,3-thiadiazole-7-carbonylthiomethyl]-1-phenylcyclopropane;
3-[benzo-1,2,3-thiadiazole-7-carbonyloxymethyl]-1-phenyl-1-propene;
2-[benzo-1,2,3-thiadiazole-7-carbonyloxy]-1-phenyl-n-propane;
2-[benzo-1,2,3-thiadiazole-7-carbonyloxy]-1-phenyl-ethanol;
2-[benzo-1,2,3-thiadiazole-7-carbonyloxy]-1-phenyl-propionic acid methyl ester;
1-[benzo-1,2,3-thiadiazole-7-carbonyloxy]-2-phenyl-n-propane;
1-[benzo-1,2,3-thiadiazole-7-carbonyloxy]-2-phenyl-n-butane;
1-[benzo-1,2,3-thiadiazole-7-carbonyloxy]-2-(2',4'-dichlorophenyl)-n-butane;
1-[6-fluorobenzo-1,2,3-thiadiazole-7-carbonyloxy]-2-(2',4'-dichlorophenyl)-n-butane.

8. A composition for protecting plants against attack by microorganisms that comprises as active component at least one compound according to claim 1 together with customary carriers and adjuvants.

9. A composition according to claim 8 that comprises as active component at least one compound according to claims 2 to 6.

10. A composition according to claim 8 that comprises as active component a compound of formula I according to claim 7.

11. A process for the preparation of an agrochemical composition as claimed in claim 8 which comprises homogeneously mixing at least one compound defined in accordance with claim 1 with suitable solid or liquid carriers and adjuvants.

12. The use of a compound according to claim 1 for protecting plants against attack by phytopathogenic microorganisms.

13. The use of a compound according to any one of claims 2 to 7 for protecting plants against attack by phytopathogenic microorganisms.

14. A method of protecting plants against attack by phytopathogenic microorganisms which comprises applying as active ingredient to the plant or the locus thereof a compound according to claim 1.

15. A method of protecting plants against attack by phytopathogenic microorganisms which comprises applying as active ingredient to the plant or the locus thereof a compound according to any one of claims 2 to 7.

16. A method according to claims 14 and 15 wherein the phytopathogenic microorganisms are fungal organisms.

## Revendications

**Revendications pour les Etats contractants suivants : AT, BE, CH, DE, DK, FR, GB, GR, IT, LI, LU, NL, SE**

1. Composés de formule I

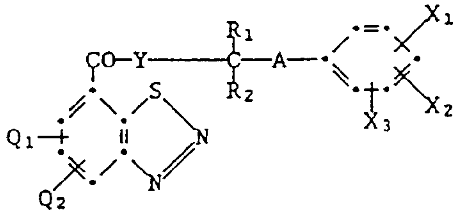

(I)

dans laquelle

| | |
|---|---|
| Y | représente l'oxygène ou le soufre; |
| $Q_1$ et $Q_2$ | représentent chacun, indépendamment l'un de l'autre, l'hydrogène ou un halogène; |
| $R_1$ et $R_2$ | représentent chacun , indépendamment l'un de l'autre, l'hydrogène ou un groupe alkyle en C 1-C 4; |
| $X_1$ | représente l'hydrogène, un halogène, un groupe alkyle en C 1-C 4, trifluorométhyle, alkyle en C 1-C 3, alcoxy en C 1-C 2 substitué par des halogènes dans la partie alkyle, nitro, cyano, diméthyiamino, phényle, phénoxy, phényle ou phénoxy substitué par des halogènes et/ou des groupes alkyle en C 1-C 2 et/ou alcoxy en C 1-C 2; |
| $X_2$ | représente l'hydrogène, un halogène ou un groupe méthyle; |
| $X_3$ | représente l'hydrogène ou un halogène; |
| | et |
| A | représente les ponts |

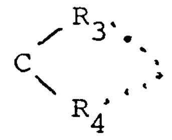 , 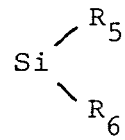 ,

carbonyle, éthénylène ou éthynylène;

dans lesquels

$R_3$ représente un groupe alkyle en C 1-C 6, alcényle en C 3-C 6, hydroxy, alcoxy en C 1-C 6, O-C(O)-alkyle en C 1-C 4, carboxyle, COO-alkyle en C 1-C 4, cyano ou l'hydrogène sous réserve que, lorsque $R_3$ représente l'hydrogène, $R_1$, $R_2$ et $R_4$ ne peuvent représenter simultanément l'hydrogène;

$R_4$ représente un groupe alkyle en C 1-C 6, alcoxy en C 1-C 6 ou l'hydrogène, sous réserve que, lorsque $R_4$ représente l'hydrogène, $R_1$, $R_2$ et $R_3$ ne peuvent représenter simultanément l'hydrogène;

$R_5$ représente un groupe alkyle en C 1-C 6, cycloalkyle en C 3-C 6, alcoxy en C 1-C 6, phényle, ou phényle substitué par des halogènes ou des groupes méthoxy;

$R_6$ représente un groupe alkyle en C 1-C 6 ou alcoxy en C 1-C 6;

ou encore le pont

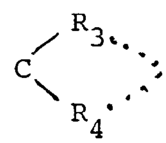

qui peut consister en un carbocycle saturé ou insaturé de 3 à 7 chaînons $W_1$ ou en un hétérocycle saturé ou insaturé de 5 à 7 chaînons $W_2$ contenant 1 ou 2 hétéroatomes choisis parmi l'oxygène, le soufre ou l'azote;

ou bien le pont

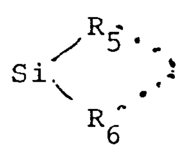

qui consiste en un hétérocycle saturé ou insaturé de 3 à 7 chaînons $W_3$ contenant au maximum, en tant qu'autres hétéroatomes, 2 atomes d'oxygène ou de soufre.

**2.** Composés de formule I selon revendication 1, pour lesquels :

Y représente l'oxygène ou le soufre;

$Q_1$ et $Q_2$ représentent chacun, indépendamment l'un de l'autre, l'hydrogène ou un halogène;

$R_1$ et $R_2$ représentent chacun, indépendamment l'un de l'autre, l'hydrogène ou un groupe alkyle en C 1-C 4;

$X_1$ représente l'hydrogène, un halogène, un groupe alkyle en C 1-C 4, trifluorométhyle, alkyle en C 1-C 3, alcoxy en C 1-C 2 substitué par des halogènes dans la partie alkyle, nitro, cyano, diméthylamino, phényle, phénoxy, phényle ou phénoxy substitué par des halogènes et/ou des groupes alkyle en C 1-C 2 et/ou alcoxy en C 1-C 2;

$X_2$ représente l'hydrogène, un halogène ou un groupe méthyle;

$X_3$ représente l'hydrogène ou un halogène; et

A représente les ponts

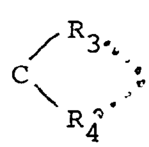 ,

carbonyle, éthénylène ou éthynylène; dans lesquels

$R_3$ représente un groupe alkyle en C 1-C 6, alcényle en C 3-C 6, hydroxy, alcoxy en C 1-C 6, O-C(O)-alkyle en C 1-C 4, carboxyle, COO-alkyle en C 1-C 4, cyano ou l'hydrogène, sous réserve que, lorsque $R_3$ représente l'hydrogène, $R_1$, $R_2$ et $R_4$ ne peuvent représenter simultanément l'hydrogène;

$R_4$ représente un groupe alkyle en C 1-C 6, alcoxy en C 1-C 6 ou l'hydrogène, sous réserve que lorsque $R_4$ représente l'hydrogène, $R_1$, $R_2$ et $R_3$, ne peuvent représenter simultanément l'hydrogène;

ou encore le pont

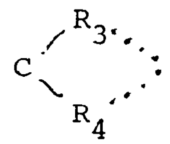

qui peut également consister en un carbocycle saturé ou insaturé de 3 à 7 chaînons $W_1$ ou en un hétérocycle saturé ou insaturé de 5 à 7 chaînons $W_2$ contenant 1 ou 2 hétéroatomes choisis parmi l'oxygène, le soufre ou l'azote.

**3.** Composés de formule I de la revendication 1 dans laquelle :

Y représente l'oxygène;

$Q_1$ et $Q_2$ représentent chacun, indépendamment l'un de l'autre, l'hydrogène, le fluor, le chlore ou le brome;

$R_1$ et $R_2$ représentent chacun, indépendamment l'un de l'autre, l'hydrogène, un groupe méthyle ou éthyle;

$X_1$ représente l'hydrogène, un halogène, un groupe alkyle en C 1-C 4, alcoxy en C 1-C 3, phényle, phénoxy, phényle substitué par des halogènes ou des groupes alkyle en C 1-C 2 ou phénoxy substitué;

$X_2$ représente l'hydrogène ou un halogène;

$X_3$ représente l'hydrogène;

A représente les ponts

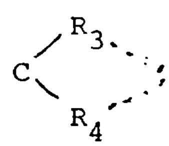 ,

éthénylène, éthynylène ou carbonyle;

$R_3$ représente un groupe alkyle en C 1-C 6, alcényle en C 3-C 6, hydroxy, alcoxy en C 1-C 6, cyano ou COO-alkyle en C 1-C 3;

$R_4$ représente l'hydrogène, un groupe méthyle, éthyle, méthoxy ou éthoxy;

$W_1$ représente un groupe cycloheptyle, cyclohexyle, cyclopentyle, cyclobutyle ou cyclopropyle;

$W_2$ représente un groupe 1,3-dioxolanne-2-yle, 1,3-dioxanne-2-yle, 1,3-dioxolanne-4-one-5-yle, tétrahydrofuranne-2-one-5-yle, 1,3-dithianne-2-yle, tétrahydro-1,3-oxazine-2-yle ou oxazolidine-2-yle.

**4.** Composés de formule I de la revendication 1, dans laquelle :

Y représente l'oxygène;

$Q_1$ et $Q_2$ représentent chacun, indépendamment l'un de l'autre, l'hydrogène ou le fluor;

$R_1$ et $R_2$ représentent l'hydrogène ou un groupe méthyle;

$X_1$ représente l'hydrogène, un halogène, un groupe méthyle, éthyle, méthoxy, éthoxy, phényle, phénoxy, phényle substitué par le fluor, le chlore, un groupe méthyle ou éthyle ou phénoxy substitué;

$X_2$ représente l'hydrogène ou un halogène,

$X_3$ représente l'hydrogène;

A représente les ponts

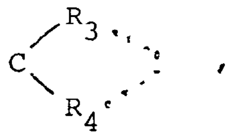

éthénylène ou éthynylène;

$R_3$ représente un groupe alkyle en C 2-C 4, alcényle en C 3-C 6, hydroxy, O-alkyle en C 1-C 4, cyano ou COO-alkyle en C 1-C 3;

$R_4$ représente l'hydrogène, un groupe méthyle, éthyle, méthoxy ou éthoxy;

$W_1$ représente un groupe cyclopentyle ou cyclohexyle;

$W_2$ représente un groupe 1,3-dioxolanne-2-yle, 1,3-dioxanne-2-yle, 1,3-dioxolanne-4-one-5-yle, tétrahydrofuranne-2-one-5-yle, 1,3-dithianne-2-yle, tétrahydro-1,3-oxazine-2-yle ou oxazolidine-2-yle.

**5.** Composés de formule I de la revendication 1, dans laquelle :

Y représente l'oxygène;

$Q_1$ et $Q_2$ représentent l'hydrogène;

$R_1$ et $R_2$ représentent l'hydrogène;

$X_1$ représente l'hydrogène, le fluor, le chlore, un groupe méthyle, méthoxy, phényle, phénoxy, phényle substitué par le fluor ou des groupes méthyle ou phénoxy substitué;

$X_2$ représente l'hydrogène, le fluor ou le chlore;

$X_3$ représente l'hydrogène;

A représente les ponts

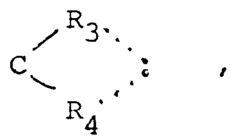

éthénylène ou éthynylène;

$R_3$ représente un groupe méthyle, éthyle, allyle, hydroxy, méthoxy ou éthoxy;

$R_4$ représente l'hydrogène, un groupe méthyle, éthyle, méthoxy ou éthoxy;

$W_1$ représente un groupe cyclopentyle ou cyclohexyle;

$W_2$ représente un groupe 1,3-dioxolanne-2-yle, 1,3-dioxanne-2-yle, 1,3-dioxolanne-4-one-5-yle, tétrahydrofuranne-2-one-5-yle, 1,3-dithianne-2-yle, tétrahydro-1,3-oxazine-2-yle ou oxazolidine-2-yle.

**6.** Composés de formule 1 de la revendication 1, dans laquelle :

Y représente l'oxygène;

$Q_1$ et $Q_2$ représentent l'hydrogène;

$R_1$ et $R_2$ représentent l'hydrogène;

$X_1$ représente l'hydrogène, le fluor, le chlore ou un groupe méthyle;

$X_2$ représente l'hydrogène, le fluor ou le chlore;

$X_3$ représente l'hydrogène;

A représente les ponts

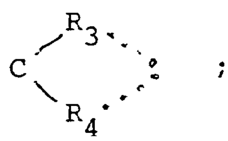

$R_3$ représente un groupe méthyle, éthyle, n-propyle, isopropyle, n-butyle, allyle, méthoxy ou éthoxy;

$R_4$ représente l'hydrogène, un groupe méthyle ou éthyle.

**7.** Un composé de formule I de la revendication 1, pris dans le groupe suivant : [benzo-1,2,3-thiadiazole-7-carbonyloxyméthyl]-2',4'-dichlorophénylcétone; 2-[benzo-1,2,3-thiadiazole-7-carbonyloxyméthyl]-1-phényl-cyclopropane; 1-[benzo-1,2,3-thiadiazole-7-carbonyloxyméthyl]-1-(4-méthoxyphényl)-cyclopentane; 2-[benzo-1,2,3-thiadiazole-7-carbonylthiométhyl]-1-phényl-cyclopropane; 3-[benzo-1,2,3-thiadiazole-7-carbonyloxyméthyl]-1-phényl-1-propène; 2-[benzo-1,2,3-thiadiazole-7-carbonyloxy]-1-phényl-n-propane; 2-[benzo-1,2,3-thiadiazole-7-carbonyloxy]-1-phényl-éthanol; 2-[benzo-1,2,3-thiadiazole-7-carbonyloxy]-1-phényl-propionate de méthyle; 1-[benzo-1,2,3-thiadiazole-7-carbonyloxy]-2-phényl-n-propane; 1-[benzo-1,2,3-thiadiazole-7-carbonyloxy]-2-phényl-n-butane; 1-[benzo-1,2,3-thiadiazole-7-carbonyloxy]-2-(2,4'-dichiorophényl)-n-butane; 1-[6-fluoro-benzo-1,2,3-thiadiazole-7-carbonyloxy]-2-(2',4'-dichlorophényl)-n-butane.

**8.** Procédé de préparation des composés de formule I de la revendication 1, caractérisé en ce que l'on fait réagir

a) un dérivé activé d'acide carboxylique de formule II

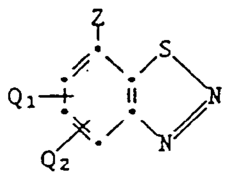

(II)

avec un alcool ou thiol de formule III

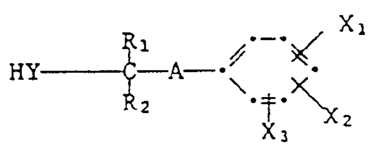

(III)

en présence d'une base, éventuellement en présence d'un catalyseur dans des solvants inertes à des températures de -20 à 160°C, Z représentant les groupes Hal-Co

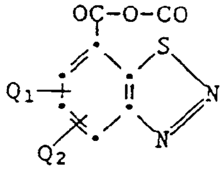

ou

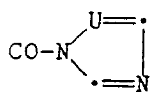

U représentant CH ou N et Hal un halogène;

b) l'acide libre de formule II'

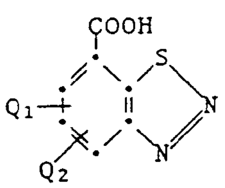

(II')

avec un alcool ou thiol de formule III en présence d'un catalyseur acide dans un solvant inerte ou dans un excès de l'alcool ou thiol de formule III utilisé pour l'estérification, à des températures de 10 à 180°C, les radicaux et symboles A, $Q_1$, $Q_2$, $R_1$, $R_2$, $X_1$, $X_2$, $X_3$ et Y ayant les significations indiquées en référence à la formule I.

9. Produit pour protéger les végétaux contre l'attaque par des microorganismes, caractérisé en ce qu'il contient, avec des véhicules et produits auxiliaires usuels, au moins un composant actif consistant en un composé selon revendication 1 .

10. Produit selon revendication 9, caractérisé en ce qu'il contient au moins un composant actif consistant en un composé selon les revendications 2 à 6.

11. Produit selon revendication 9, caractérisé en ce qu'il contient en tant que composant actif le composé de formule I de la revendication 7.

12. Procédé de préparation d'un produit agro-chimique selon revendication 9, caractérisé en ce que l'on mélange intimement au moins un composé tel que défini dans la revendication 1 avec des véhicules et produits auxiliaires solides ou liquides appropriés.

13. Utilisation des composés selon revendication 1 pour la protection des végétaux contre l'attaque par des microorganismes phytopathogènes.

14. Utilisation des composés selon une des revendications 2 à 7 pour la protection des végétaux contre l'attaque par des microorganismes phytopathogènes.

15. Procédé pour protéger les végétaux contre l'attaque par des microorganismes phytopathogènes, caractérisé en ce que l'on applique sur la plante ou son habitat une substance active consistant en -un composé selon revendication 1.

16. Procédé pour protéger les végétaux contre l'attaque par des microorganismes phytopathogènes, caractérisé en ce que l'on applique sur la plante ou son habitat une substance active consistant en un composé selon l'une des revendications 2 à 7.

17. Procédé selon les revendications 15 et 16, caractérisé en ce que les microorganismes phytopathogènes sont des mycètes.

**Revendications pour l'Etat contractant suivant : ES**

1. Procédé de préparation des composés de formule I

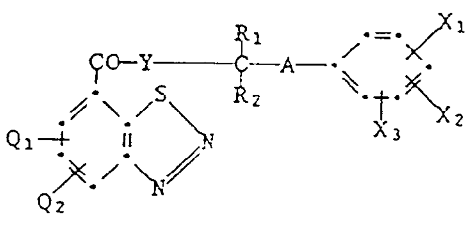

(I)

dans laquelle

| | |
|---|---|
| Y | représente l'oxygène ou le soufre; |
| $Q_1$ et $Q_2$ | représentent chacun, indépendamment l'un de l'autre, l'hydrogène ou un halogène; |
| $R_1$ et $R_2$ | représentent chacun, indépendamment l'un de l'autre, l'hydrogène ou un groupe alkyle en C 1-C 4; |
| $X_1$ | représente l'hydrogène, un halogène, un groupe alkyle en C 1-C 4, trifluorométhyle, alkyle en C 1-C 3, alcoxy en C 1-C 2 substitué par des halogènes dans la partie alkyle, nitro, cyano, diméthylamino, phényle, phénoxy, phényle ou phénoxy substitué par des halogènes et/ou des groupes alkyle en C 1-C 2 et/ou alcoxy en C 1-C 2; |
| $X_2$ | représente l'hydrogène, un halogène ou un groupe méthyle; |
| $X_3$ | représente l'hydrogène ou un halogène; |

et

A représente les ponts

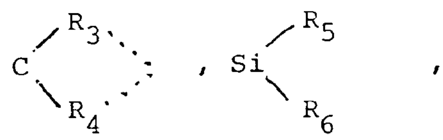

carbonyle, éthénylène ou éthynylène;

dans lesquels

$R_3$ représente un groupe alkyle en C 1-C 6, alcényle en C 3-C 6, hydroxy, alcoxy en C 1-C 6, O-C(O)-alkyle en C 1-C 4, carboxyle, COO-alkyle en C 1-C 4, cyano ou l'hydrogène sous réserve que, lorsque $R_3$ représente l'hydrogène, $R_1$, $R_2$ et $R_3$ ne peuvent représenter simultanément l'hydrogène;

$R_4$ représente un groupe alkyle en C 1-C 6, alcoxy en C 1-C 6 ou l'hydrogène, sous réserve que, lorsque $R_4$ représente l'hydrogène, $R_1$, $R_2$ et $R_3$ ne peuvent représenter simultanément l'hydrogène;

$R_5$ représente un groupe alkyle en C 1-C 6, cycloalkyle en C 3-C 6, alcoxy en C 1-C 6, phényle, ou phényle substitué par des halogènes ou des groupes méthoxy;

$R_6$ représente un groupe alkyle en C 1-C 6 ou alcoxy en C 1-C 6;

ou encore le pont

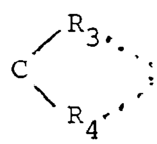

qui peut consister en un carbocycle saturé ou insaturé de 3 à 7 chaînons $W_1$ ou en un hétérocycle saturé ou insaturé de 5 à 7 chaînons $W_2$ contenant 1 ou 2 hétéroatomes choisis parmi l'oxygène, le soufre ou l'azote;

ou bien le pont

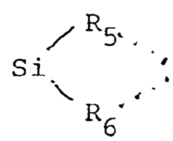

qui consiste en un hétérocycle saturé ou insaturé de 3 à 7 chaînons $W_3$ contenant au maximum, en tant qu'autres hétéroatomes, 2 atomes d'oxygène ou de soufre,

caractérisé en ce que l'on fait réagir :

a) un dérivé activé d'acide carboxylique de formule II

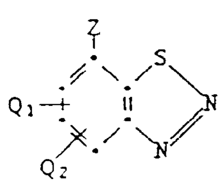

(II)

avec un alcool ou thiol de formule III

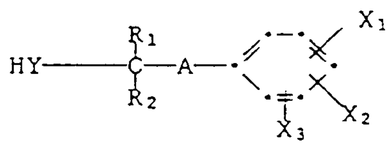

(III)

en présence d'une base, éventuellement en présence d'un catalyseur dans des solvants inertes à des températures de -20 à 160°C, Z représentant les groupes Hal-Co

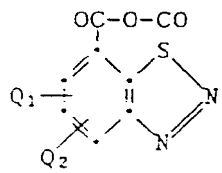

ou

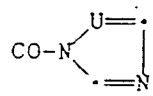

U représentant CH ou N et Hal un halogène;

b) l'acide libre de formule II'

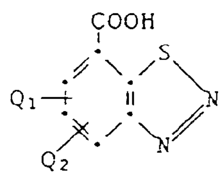

(II')

avec un alcool ou thiol de formule III en présence d'un catalyseur acide dans un solvant inerte ou dans un excès de l'alcool ou thiol de formule III utilisé pour l'estérification, à des températures de 10 à 180°C, les radicaux et symboles A, $Q_1$, $Q_2$, $R_1$, $R_2$, $X_1$, $X_2$, $X_3$ et Y ayant les significations indiquées en référence à la formule I.

**2.** Procédé de préparation des composés de formule I de la revendication 1 dans laquelle :

| | |
|---|---|
| Y | représente l'oxygène ou le soufre; |
| $Q_1$ et $Q_2$ | représentent chacun, indépendamment l'un de l'autre, l'hydrogène ou un halogène; |
| $R_1$ et $R_2$ | représentent chacun, indépendamment l'un de l'autre, l'hydrogène ou un groupe alkyle en C 1-C 4; |
| $X_1$ | représente l'hydrogène, un halogène, un groupe alkyle en C 1-C 4, trifluorométhyle, alkyle en C 1-C 3, alcoxy en C 1-C 2 substitué par des halogènes dans la partie alkyle, nitro, cyano, diméthylamino, phényle, phénoxy, phényle ou phénoxy substitué par des halogènes et/ou des groupes alkyle en C 1-C 2 et/ou alcoxy en C 1-C 2; |
| $X_2$ | représente l'hydrogène, un halogène ou un groupe méthyle; |
| $X_3$ | représente l'hydrogène ou un halogène; et |
| A | représente les ponts |

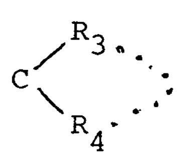

,

carbonyle, éthénylène ou éthynylène; dans lesquels

| | |
|---|---|
| $R_3$ | représente un groupe alkyle en C 1-C 6, alcényle en C 3-C 6, hydroxy, alcoxy en C 1-C 6, O-C(O)-alkyle en C 1-C 4, carboxyle, COO-alkyle en C 1-C 4, cyano ou l'hydrogène, sous réserve que, lorsque $R_3$ représente l'hydrogène, $R_1$, $R_2$ et $R_4$ ne peuvent représenter simultanément l'hydrogène; |
| $R_4$ | représente un groupe alkyle en C 1-C 6, alcoxy en C 1-C 6 ou l'hydrogène, sous réserve que lorsque $R_4$ représente l'hydrogène, $R_1$, $R_2$ et $R_3$, ne peuvent représenter simultanément l'hydrogène; |

ou encore le pont

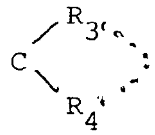

qui peut également consister en un carbocycle saturé ou insaturé de 3 à 7 chaînons $W_1$ ou en un hétérocycle saturé ou insaturé de 5 à 7 chaînons $W_2$ contenant 1 ou 2 hétéroatomes choisis parmi l'oxygène, le soufre ou l'azote.

**3.** Procédé de préparation des composés de formule I de la revendication 1 dans laquelle

| | |
|---|---|
| Y | représente l'oxygène; |
| $Q_1$ et $Q_2$ | représentent chacun, indépendamment l'un de l'autre, l'hydrogène, le fluor, le chlore ou le brome; |
| $R_1$ et $R_2$ | représentent chacun, indépendamment l'un de l'autre, l'hydrogène, un groupe méthyle ou éthyle; |
| $X_1$ | représente l'hydrogène, un halogène, un groupe alkyle en C 1-C 4, alcoxy en C 1-C 3, phényle, phénoxy, phényle substitué par des halogènes ou des groupes alkyle en C 1-C 2 ou phénoxy substitué; |
| $X_2$ | représente l'hydrogène ou un halogène; |
| $X_3$ | représente l'hydrogène; |
| A | représente les ponts |

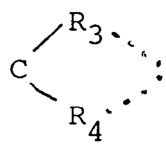 ,

éthénylène, éthynylène ou carbonyle;

| | |
|---|---|
| $R_3$ | représente un groupe alkyle en C 1-C 6, alcényle en C 3-C 6, hydroxy, alcoxy en C 1-C 6, cyano ou COO-alkyle en C 1-C 3; |
| $R_4$ | représente l'hydrogène, un groupe méthyle, éthyle, méthoxy ou éthoxy; |
| $W_1$ | représente un groupe cycloheptyle, cyclohexyle, cyclopentyle, cyclobutyle ou cyclopropyle; |
| $W_2$ | représente un groupe 1,3-dioxolanne-2-yle, 1,3-dioxanne-2-yle, 1,3-dioxolanne-4-one-5-yle, tétrahydrofuranne-2-one-5-yle, 1,3-dithianne-2-yle, tétrahydro-1,3-oxazine-2-yle ou oxazolidine-2-yle. |

**4.** Procédé de préparation des composés de formule I de la revendication 1, dans laquelle :

| | |
|---|---|
| Y | représente l'oxygène; |
| $Q_1$ et $Q_2$ | représentent chacun, indépendamment l'un de l'autre, l'hydrogène ou le fluor; |
| $R_1$ et $R_2$ | représentent l'hydrogène ou un groupe méthyle; |
| $X_1$ | représente l'hydrogène, un halogène, un groupe méthyle, éthyle, méthoxy, éthoxy, phényle, phénoxy, phényle substitué par le fluor, le chlore, un groupe méthyle ou éthyle ou phénoxy substitué; |
| $X_2$ | représente l'hydrogène ou un halogène; |
| $X_3$ | représente l'hydrogène; |
| A | représente les ponts |

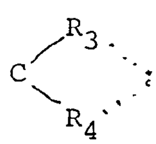 ,

éthénylène ou éthynylène;

$R_3$ représente un groupe alkyle en C 2-C 4, alcényle en C 3-C 6, hydroxy, O-alkyle en C 1-C 4, cyano ou COO-alkyle en C 1-C 3;

$R_4$ représente l'hydrogène, un groupe méthyle, éthyle, méthoxy ou éthoxy;

$W_1$ représente un groupe cyclopentyle ou cyclohexyle;

$W_2$ représente un groupe 1,3-dioxolanne-2-yle, 1,3-dioxanne-2-yle, 1,3-dioxolanne-4-one-5-yle, tétrahydrofuranne-2-one-5-yle, 1,3-dithianne-2-yle, tétrahydro-1,3-oxazine-2-yle ou oxazolidine-2-yle.

5. Procédé de préparation des composés de formule I de la revendication 1, dans laquelle :

Y représente l'oxygène;

$Q_1$ et $Q_2$ représentent l'hydrogène;

$R_1$ et $R_2$ représentent l'hydrogène;

$X_1$ représente l'hydrogène, le fluor, le chlore, un groupe méthyle, méthoxy, phényle, phénoxy, phényle substitué par le fluor ou des groupes méthyle ou phénoxy substitué;

$X_2$ représente l'hydrogène, le fluor ou le chlore;

$X_3$ représente l'hydrogène

A représente les ponts

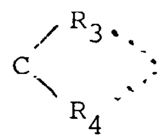

,

éthénylène ou éthynylène;

$R_3$ représente un groupe méthyle, éthyle, allyle, hydroxy, méthoxy ou éthoxy;

$R_4$ représente l'hydrogène, un groupe méthyle, éthyle, méthoxy ou éthoxy;

$W_1$ représente un groupe cyclopentyle ou cyclohexyle;

$W_2$ représente un groupe 1,3-dioxolanne-2-yle, 1,3-dioxanne-2-yle, 1,3-dioxolanne-4-one-5-yle, tétrahydrofuranne-2-one-5-yle, 1,3-dithianne-2-yle, tétrahydro-1,3-oxazine-2-yle ou oxazolidine-2-yle.

6. Procédé de préparation des composés de formule I de la revendication 1, dans laquelle

Y représente l'oxygène;

$Q_1$ et $Q_2$ représentent l'hydrogène;

$R_1$ et $R_2$ représentent l'hydrogène;

$X_1$ représente l'hydrogène, le fluor, le chlore ou un groupe méthyle;

$X_2$ représente l'hydrogène, le fluor ou le chlore;

$X_3$ représente l'hydrogène;

A représente les ponts

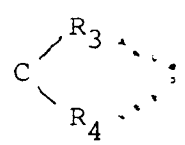

;

$R_3$ représente un groupe méthyle, éthyle, n-propyle, isopropyle, n-butyle, allyle, méthoxy ou éthoxy;

$R_4$ représente l'hydrogène, un groupe méthyle ou éthyle.

7. Procédé de préparation d'un composé de formule I de la revendication 1, pris dans le groupe suivant :

[benzo-1,2,3-thiadiazole-7-carbonyloxyméthyl]-2',4'-dichlorophénylcétone;

2-[benzo-1,2,3-thiadiazole-7-carbonyloxyméthyl]-1-phényl-cyclopropane;

1-[benzo-1,2,3-thiadiazole-7-carbonyloxyméthyl]-1-(4-méthoxyphényl)-cyclopentane;

2-[benzo-1,2,3-thiadiazole-7-carbonylthiométhyl]-1-phénylcyclopropane;

3-[benzo-1,2,3-thiadiazole-7-carbonyloxyméthyl]-1-phényl-1-propène;

2-[benzo-1,2,3-thiadiazole-7-carbonyloxy]-1-phényl-n-propane;
2-[benzo-1,2,3-thiadiazole-7-carbonyloxy]-1-phényl-éthanol;
2-[benzo-1,2,3-thiadiazole-7-carbonyloxy]-1-phényl-propionate de méthyle;
1-[benzo-1,2,3-thiadiazole-7-carbonyloxy]-2-phényl-n-propane;
1-[benzo-1,2,3-thiadiazole-7-carbonyloxy]-2-phényl-n- butane;
1-[benzo-1,2,3-thiadiazole-7-carbonyloxy]-2-(2',4'-dichlorophényl)-n-butane;
1-[6-fluoro-benzo-1,2,3-thiadiazole-7-carbonyloxy]-2-(2',14'-dichlorophényl)-n-butane.

**8.** Produit pour protéger les végétaux contre l'attaque par des microorganismes, caractérisé en ce qu'il contient, avec des véhicules et produits auxiliaires usuels, au moins un composant actif consistant en un composé selon revendication 1.

**9.** Produit selon revendication 8, caractérisé en ce qu'il contient au moins un composant actif consistant en un composé selon les revendications 2 à 6.

**10.** Produit selon revendication 8, caractérisé en ce qu'il contient en tant que composant actif le composé de formule I de la revendication 7.

**11.** Procédé de préparation d'un produit agro-chimique selon revendication 8, caractérisé en ce que l'on mélange intimement au moins un composé tel que défini dans la revendication 1 avec des véhicules et produits auxiliaires solides ou liquides appropriés.

**12.** Utilisation des composés selon revendication 1 pour la protection des végétaux contre l'attaque par des microorganismes phytopathogènes.

**13.** Utilisation des composés selon une des revendications 2 à 7 pour la protection des végétaux contre l'attaque par des microorganismes phytopathogènes.

**14.** Procédé pour protéger les végétaux contre l'attaque par des microorganismes phytopathogènes, caractérisé en ce que l'on applique sur la plante ou son habitat une substance active consistant en un composé elon revendication 1.

**15.** Procédé pour protéger les végétaux contre l'attaque par des microorganismes phytopathogènes, caractérisé en ce que l'on applique sur la plante ou son habitat une substance active consistant en un composé selon l'une des revendications 2 à 7.

**16.** Procédé selon les revendications 14 et 15, caractérisé en ce que les microorganismes phytopathogènes sont des mycètes.